# EUROPÄISCHE PATENTANMELDUNG

(11) **EP 4 307 309 A1**
(43) Veröffentlichungstag der Anmeldung: **17.01.2024**
(21) Anmeldenummer: 22184348.5
(22) Anmeldetag: 12.07.2022
(51) Int. Cl.: G16H 10/40, G16H 40/67, G16H 50/70

(54) **SYSTEM, VERFAHREN UND ANORDNUNGEN FÜR DAS INTELLIGENTE ERSTELLEN VON AUSGABEN AUFGRUND VON LABORWERTEN UND PATIENTENMETADATEN IN DOKUMENTEN**

(71) Anmelder: iunera GmbH & Co. KG, 69190 Walldorf Baden-Wuerttemberg (DE)
(72) Erfinder: Frey, Tim, 74855 Haßmersheim (DE)

(57) **Zusammenfassung**

Die vorliegende Erfindung betrifft ein System oder Anordnungen und/oder Verfahren, für das Erstellen von Ausgaben (190) aufgrund von Dokumenten, die Laborwerte (110) und Patientenmetainformationen (100) beinhalten, wobei ein System, Verfahren oder eine Anordnung zumindest folgende Merkmale aufweisen:
a. Zumindest eine Speichereinheit (120), die zumindest eine Rahmenbedingung (200) RB und hierzu zumindest einen verknüpften Referenzbereich (130) von Laborwerten (110) enthält;
b. Zumindest ein Laborwert (110) und zumindest ein Patientenmetadatum (100), das über zumindest ein Computernetzwerk (220) übertragen wird;
c. Zumindest ein übertragenes Patientenmetadatum (100), verwendet wird um zumindest einen Referenzbereich (130) R aus einer Speichereinheit (120) zu bestimmen, indem die Übereinstimmung zumindest einer Rahmenbedingung (200) RB und dem Patientenmetadatum (100) geprüft wird;
d. Zumindest eine Bewertung B von zumindest einem Laborwert (110) aus b. unter der Einbeziehung von zumindest einem Referenzbereich (130) R;
e. Ausgabe zumindest einer Bewertung B.

## Beschreibung

### 1. Technisches Gebiet

Die vorliegende Erfindung 180 betrifft Systeme, Verfahren und Anordnungen für das intelligente Erstellen von Hinweisen aufgrund von Laborwerten 110 durch die spezielle Bewertung durch Patientenmetadaten 100. Hierbei wurde die Erfindung 180 unter dem besonderen Augenmerk gemacht, dass Laborwerte 110 oftmals in Datenbanken gespeichert werden. Diese Datenbanken können ohne menschliches Zutun oder Eingriff am menschlichen Körper ausgewertet werden. Insbesondere umfasst die Erfindung 180 daher insbesondere solche Systeme, Verfahren und Anordnungen, bei denen Dokumente über eines oder mehrere mobile Computernetzwerke 220 ausgetauscht werden. Dabei wurde die Erfindung 180 besonders unter Betrachtung von Geodaten 999 und gleichzeitigem Aufdecken von relevanten Geoinformationen und deren Zusammenhang mit Patientenmetadaten 100 gemacht.

### 2. Stand der Technik

Das Messen von Laborwerten 110 ist Stand der Technik. Patienten bekommen Blut entnommen oder geben Urin ab. Die Flüssigkeiten werden dann technisch auf das Vorhandensein von Biomarkern, wie Tumormarkern, Substanzen oder weiteren Eigenschaften überprüft. Diese Eigenschaften werden gemeinhin als Blutwerte bezeichnet, weshalb wir in dieser Anmeldung keine Unterscheidung zwischen Biomarkern, Eigenschaften, Blutwerten oder Laborwerten 110 oder der Mehrzahl dieser machen.

Oftmals werden Laborwerte 110 gesammelt, elektronisch über Computernetzwerke 220 übertragen und in Datenbanken gespeichert oder eine Sammlung von Laborwerten 110 wird als Laborbefund 110 analog digital an den Arzt oder Patienten zugestellt.

Der Laborbefund zeigt neben den ermittelten Werten die geltenden Referenzwerte 130, also den Normalbereich, an. Individuelle Abweichungen vom Referenzbereich 130 können ein Hinweis auf eine sich entwickelnde oder bereits manifeste Erkrankung sein. Aber nicht jede kleine Abweichung bedeutet notwendigerweise eine gesundheitliche Störung. Zum Einen können Laborwerte 110 schwanken, zum Anderen zieht der Arzt für eine genaue Diagnose immer auch den Verlauf der persönlichen Werte aus verschiedenen Messungen, den körperlichen Gesamtzustand und die im ausführlichen Patientengespräch erhobenen Befunde hinzu. Die Laborwerte 110 allein sagen also nicht allzuviel aus, da die Bedingungen bei jedem Patienten anders sind und die Referenzbereiche 130 dadurch schwanken. Ein Beispiel hierzu ist:
https://www.pronovabkk.de/leben/blutbild-und-blutwerte.html

Oftmals werden dann Laborwerte 110 anhand einer Skala überprüft und in der Software oder einem Ausdruck bei Auffälligkeiten annotiert. Beispielsweise liegt ein Schilddrüsenhormonwert TSH normalerweise in einem Bereich zwischen 0,4 und 4,0 für einen Erwachsenen. Sofern der Wert 250 nun unter- oder oberhalb dieses Referenzbereichs 130 liegen sollte, wird der Wert 250 z. B. fett gedruckt oder anderweitig markiert, damit der Arzt darauf aufmerksam gemacht wird. Dies führt dazu, dass Ärzte im Regelfall Blutwerte nach markierten Anomalien durchsuchen.

Oftmals werden jedoch solche Systeme, die Abweichungen zu Referenzbereichen 130 beurteilen, in Europa und Nordamerika erforscht und entwickelt, wo z. B. ein starker Winter vorherrscht. Dies führt z. B. oftmals zu einem leichten Vitamin D Defizit bei Personen im Winter. Die Referenzbereiche 130 sind im Regelfall an diese Umstände angepasst. Sofern nun ein "nordeuropäischer Referenzbereich 130" bei Personen verwendet wird, die gerade in einem tropischen Land gelebt und zurückgekehrt sind und trotz dessen niedrige Vitamin D Werte haben, kann die Interpretation daher im Referenzbereich 130 natürlich falsch sein, da eine solche Person weitaus höhere Sonneneinstrahlung erfahren hat. Ein Arzt kann diesen Umstand dann leicht übersehen, da durch die "Nichtexistenz einer Markierung" kein Hinweis für genaue Rückfragen gegeben ist.

Ein weiteres Beispiel ist eine Infektion mit dem Dengue Fieber oder Malaria in einem tropischen Urlaubsland, die erst nach der Rückkehr ausbricht. Wird es dem zu behandelnden Arzt nicht mitgeteilt, dass ein Patient in den Tropen war, dann ist die Wahrscheinlichkeit sehr gering, dass in Europa oder Nordamerika ein Malaria- oder Dengue Laborwert 110 ermittelt wird. Selbst bei anderen stark irregulären Blutwerten wird dann kein entsprechender Laborwert 110 ermittelt, da es für normale Mediziner nicht naheliegend ist.

Ein weiteres Problem ist, dass oftmals Laborwerte 110 für Routineuntersuchungen oder spezielle Krankheitsvermutungen erstellt werden und dann aus diesem Blickwinkel betrachtet werden. Sofern ein anderes Leiden vorliegt, in dem sich sogar in Laborwerten 110 Hinweise 190 finden könnten, wird dieses im Regelfall übersehen. Trotzdessen befinden sich oftmals die bestimmten Laborwerte 110 und die Patientenmetadaten 100 ungenutzt in einer strukturierten Datenbank (z. B. elektronische Patientenakte, Praxisverwaltungssystem oder Krankenhausverwaltungssystem), bei der sie nicht weiter als zur historischen Aufzeichnung verwendet werden.

Oftmals ergeben sich weiterhin Diagnosen von Krankheiten aus verschiedenen Faktoren, bei denen sich die Blutwerte, einzeln gesehen, alle noch knapp oder leicht über den Referenzbereichen 130 befinden, aber gemeinsam betrachtet das Krankheitsbild eindeutig ist. Ein Beispiel hierfür ist Hyperparathyreoidismus, der oftmals durch Wucherungen in den Nebenschilddrüsen ausgelöst wird. Patienten leiden oft jahrelang (z.B: 20 Jahre) unter der Krankheit bis sie einen Spezialisten finden, der dann mehrere Blutwerte in ihrer Gemeinsamkeit betrachtet oder bis sich nach Jahren einzelne Blutwerte aus den Referenzwerten 130 hinaus verschieben und sich der Umstand genauer angeschaut wird. Oftmals machen diese Patienten über Jahre hinweg viele Blutuntersuchungen durch, die sich einzeln betrachtet noch im allgemeinen Referenzbereichen 130 befinden und somit zu keiner Diagnose führen und fortfolgend mehr oder weniger ungenutzt in einer Datenbank zur historischen Datenaufzeichnung abgelegt werden.

Der Umstand von unentdeckten Krankheiten führt sogar zu speziellen Apps für die Diagnose einzelner Krankheiten. Z. B. gibt es sogar eine spezielle Hyperparathyreoidismus App, in der ein Patient manuell seine Blutwerte eintragen kann. Diese App gibt dann Hinweise darauf, wie wahrscheinlich ein Patient an Hyperparathyreoidismus leidet. Das Grundproblem ist jedoch, dass ein Patient selbst die Idee haben muss, dass er potentiell an Hyperparathyreoidismus leidet und aufgrund seiner eigenen Theorie die App herunterladen und befüllen muss.

Für andere Krankheiten würde wieder eine andere App benötigt. Somit wäre von Patienten ein vollständiges medizinisches Wissen gefordert, welche Werte zu welchen Hypothesen über welche Krankheiten führen könnten, um jeweils "die richtige App" zu suchen. Da zu sehen ist, dass schon Experten selbst die Krankheit Hyperparathyreoidismus oftmals nicht entdecken, zeigt, dass dies derzeit unmöglich ist.

Dies zeigt sehr deutlich das Hauptproblem am Stand der Technik: Laborwerte 110 werden aufgrund von ärztlichen Annahmen bestimmt, dann über Computernetzwerke 220 übertragen und befinden sich danach in technischen Datenbanken. Komplizierte Multi-Faktor Krankheiten werden nur gefunden, wenn absolute Experten durch Zufall die Laborwerte 110 betrachten. Experten überschauen aber oftmals nur ein Fachgebiet, sodass Indikatoren außerhalb des Fachgebietes im Regelfall übersehen werden.

Aus Gründen der besseren Lesbarkeit wird bei Personenbezeichnungen und personenbezogenen Hauptwörtern auf dieser Anmeldung die mannliche Form verwendet. Entsprechende Begriffe gelten grundsätzlich für alle Geschlechter oder das passende Geschlecht, wenn z.B. von Schwangerschaft geredet wird, was nur von einem Geschlecht durchlaufen werden kann.

### 3. Zusammenfassung der Erfindung

1.
   Die vorliegende Erfindung 180 löst die oben beschriebenen Probleme. In einer Ausführungsform kann die eine Variante der Erfindung die folgenden Merkmale aufweisen:
   a. Zumindest eine Speichereinheit 120, die zumindest eine Rahmenbedingung 200 RB und hierzu zumindest einen verknüpften Referenzbereich 130 von Laborwerten 110 enthält;
   b. Zumindest ein Laborwert 110 und zumindest ein Patientenmetadatum 100, das über zumindest ein Computernetzwerk 220 übertragen wird;
   c. Zumindest ein übertragenes Patientenmetadatum 100, verwendet wird um zumindest einen Referenzbereich 130 R aus einer Speichereinheit 120 zu bestimmen, indem die Übereinstimmung zumindest einer Rahmenbedingung 200 RB und dem Patientenmetadatum 100 geprüft wird;
   d. Zumindest eine Bewertung B von zumindest einem Laborwert 110 aus b. unter der Einbeziehung von zumindest einem Referenzbereich 130 R;
   e. Ausgabe zumindest einer Bewertung B.

Es ist dabei besonders hervorzuheben, dass die Laborwerte 110 insbesondere nicht als Bestandteil einer speziellen Untersuchung zu sehen sind und die Erfindung 180 sehr vorteilhaft nicht zwingend die Anwesenheit eines menschlichen Körpers begründet. Vielmehr zielt die Erfindung 180 darauf ab, dass die Laborwerte 110 und Patientenmetadaten 100 ohnehin z. B. in einem Dokument, Automat oder einer Datenbank vorhanden sind oder in einem oder mehreren Dokumenten übermittelt werden. Insbesondere Historische Daten können auf diese Art und Weise aufbereitet werden.

Somit ist die Erfindung 180 nicht als herkömmliches Diagnoseverfahren zu sehen, sondern sie verfolgt das Ziel Leiden rein technisch aufzudecken, ohne das eine Wechselwirkung mit dem menschlichen Körpers vorausgesetzt wird. Insbesondere kann die Erfindung 180 dazu benutzt werden, um Datenbanken mit Patientenmetadaten 100 zu untersuchen und somit Leiden festzustellen, die vorher unbekannt waren. Durch diese Besonderheit sind nicht alle medizinischen Schritte eines Diagnostizierverfahren vorhanden. Die Untersuchungsphase entfällt durch die Erfindung 180, da diese ein reiner datenverarbeitender Schritt ist und das Bestimmen der Abweichung besonders vorteilhaft durch Datenstrukturen 800 gelöst wird. Vielmehr ist die Erfindung 180 ein impliziter Nachbrenner, der ohne menschliche Wechselwirkung Ausgaben/Hinweise 190 auf Leiden und Ursachen generiert. Die Erfindung 180 ist sozusagen ein Nachjustierungsgerät, das keine Anwesenheit eines Patienten erfordert, um Sachverhalte aufzudecken, die nicht durch vorige medizinische Annahmen eines oder mehrerer Ärzte zurückzuführen sind. Dadurch, dass die Erfindung 180 rein datenbezogen auf der Basis von Dokumenten arbeitet, ist sie prädestiniert dafür seltene Krankheiten aufzudecken, die nicht vermutet wurden. Somit ist die Erfindung 180 durch ihre besondere Art und Weise der Datengewinnung, nicht Teil der Untersuchungsphase ist, sondern vielmehr eine Maschine, die besonders vorteilhaft dem Abgleich der ermittelten Daten mit Normwerten zwischengeschaltet ist und diesen Abgleich durch technische Daten und technische Hilfsmittel vorteilhaft verbessert, in dem neben den Laborwerten 110 noch weitere Datenquellen bei dem Abgleich mitbeachtet werden.

Rahmenbedingungen 200 stellen dabei in der Speichereinheit 120 abgelegt und ggf. generalisierte Patientenmetadaten 100 dar, die sich jedoch nicht zwingend auf einen speziellen Patienten beziehen müssen und welche mit weiteren Schlüsseln 240 und Werten 250, wie z.B. Laborwerten 110 und zugehörigen Referenzbereichen 130, verknüpft sind. Beispielsweise kommen Daten wie Alter in den Patientenmetadaten 100 vor. In den Rahmenbedingungen 200 kann ein solches Datum wie Alter, z.B. in eine Altersgruppe generalisiert werden und mit zugehörigen Laborwert 110 Referenzbereichen 130 verknüpft sein, sofern dies für einen Anwendungsfall besonders vorteilhaft ist. Selbstverständlich können Rahmenbedingungen 200 jedoch jedwedes Patientenmetadatum 100 darstellen und dieses somit mit weiteren Daten, wie z.B. Referenzbereichen verknüpfen.

Somit stellt die vorliegende Erfindung 180 ein System, Verfahren und eine Anordnung bereit, die es ermöglicht, verschiedene Laborwerte 110 im Zusammenhang mit Patientenmetadaten 100 zu bewerten. Diese Bewertung ist dadurch möglich, dass die Patientenmetadaten 100, die Laborwerte 110, Referenzbereiche 130 und Rahmenbedingungen 200 in technischen Datenstrukturen 800, vorhanden sind, sodass es möglich ist zugehörige Laborwerte 110, zugehörige Referenzbereiche 130 und übereinstimmende Rahmenbedingungen 200 mit den Patientenmetadaten 100 aufzudecken und nachfolgend zu bewerten.

Rahmenbedingungen 200 stellen somit eine Datenbasis 120 und/Struktur 800 und Regalbasis 90 bereit um Eingaben wie Patientenmetadaten 100 oder Nachgeführten 80 Laborwerten 110 durch die Erfindung 180 zu bewerten und Ausgaben 190 zu erzeugen.

Eine Ausgabe 190 ist z. B. ein Hinweis 190, Risiken, Handlungsempfehlungen, Handlungsanweisungen, Ursachenhinweisen, Diagnosehinweise oder auch Diagnosen. Eine Ausgabe 190 kann erfolgen, indem eine Zustellung an ein technisches Gerät, eine Verarbeitung durch ein technisches Gerät oder eine Anzeige in einem Ausdruck oder eine Markierung mitsamt Hinweis 190 in einer grafischen Anzeige erfolgt. Weiterhin kann aufgrund einer Ausgabe 190, durch zumindest eine Handlungsanweisung 190, direkt eine weitere technische Aktion, System oder ein Verfahren durchgeführt werden. Dies kann z. B. das automatisierte Bestimmen eines weiteren Laborwerts 110 durch Nachjustierungsanalysen (auch genannt Nachführungsanalysen) 80 sein und die darauffolgende Neuauswertung von Hinweisen 190, Risiken, Handlungsempfehlungen, Handlungsanweisungen und Ursachenhinweisen sein.

Damit die Ausgabe möglichst universell verwendet werden und diese auch automatisiert und in verschiedener Form weiterverarbeitet werden kann ist diese optional ein strukturiertes Dokument 800/190. In diesem wird der Ausgabetyp/Schlüssel 240 spezifiziert und diesem werden dann Wert(e) 250 zugeordnet. Im einfachsten Fall kann eine solche Ausgabe ein Json Dokument sein. Jedoch können natürlich auch andere Formate zum Einsatz kommen, in denen Schlüssel 240, Werte 250 oder mehrere Werte 250 zugeordnet werden können. Diese technische Strukturierung der Ausgabedokumente 800/190 ist dabei besonders vorteilhaft, wenn diese von Automaten/Maschinen/Programmen weiterverarbeitet wird. Beispielsweise können so Ausgaben von Handlungsanweisungen 190 klar interpretiert und durchgeführt werden. Selbstverständlich ist es möglich, dass die Ausgabe 190 nicht klassisch durch ein Dokument erfolgt, sondern dass durch den Einsatz der Erfindung 180 direkt in einer anderen Maschine die Struktur 800 in einem Programmcode realisiert wird und nicht in einem physischen Dokument.

Eine Bewertung von Laborwerten 110 unter Nutzung von Patientenmetadaten 100 kann anhand von Regeln 90, Wertbereichstabellen oder auch komplexeren Mechanismen, wie z. B. Entscheidungsbäumen oder Machine Learning Algorithmen (wie z. B. klassifizierende Algorithmen wie Support Vector Machines, Naive Bayes Classifier und ähnliche klassifizierende oder andere künstliche Intelligenz Mechanismen), geschehen.

Ein Beispiel für eine einfache Nutzung ist die Bewertung aufgrund der Patientenmetadaten 100 einer Schwangerschaft. Im Konkreten kann genau der Tag des Schwangerschaftsstarts oder konkret das Trimester der Schwangerschaft als Patientenmetadaten 100 zur Bewertung hinzugezogen werden. Die Bewertung kann dann diese Patientenmetadaten 100 verwenden, um Referenzwerte 130 aufzudecken, die z. B. in einer oder mehreren Tabellen oder einer Datenbank im der Speichereinheit 120 vorhanden sind. Beispielsweise haben Erwachsene einen Referenzwert 130 Calcium zwischen 8.7 - 10.2 mg/dL (2.18 - 2.55 mmol/L). Schwangere im 01. Trimester haben einen Referenzwert 130: 8.8 - 10.6 mg/dL /2.2 - 2.65 mmol/L. Schwangere im 02. Trimester haben einen Referenzwert 130: 8.2 - 9 mg/dL /2.05 - 2.25 mmol/L, Schwangere im 03. Trimester haben einen Referenzwert 130: 8.2 - 9.7mg/dL /2.05 - 2.43 mmol/L.

Letztendlich können durch die Erfindung 180 diese Wertebereiche dann automatisiert verwendet werden, um für die Patientenmetadaten 100 der Schwangerschaft dann die passenden gültigen Wertebereiche zu vergleichen. Ohne die Erfindung 180 erfolgt ein Calciumtest einer Schwangeren anhand von "unschwangeren" Wertebereichen und anormale Werte bleiben daher heutzutage oftmals unentdeckt.

Die Patientenmetadaten 100 werden verwendet, um den Laborwert 110 dem passenden Referenzbereich 130 zuzuordnen. Im Beispiel mit der Schwangerschaft kann nun konkret der Referenzbereich 130 durch die passenden Rahmenbedingungen 200 und übereinstimmenden Patientenmetadaten 100 des Schwangerschaftstrimesters gewählt werden. Durch dies kann fortfolgend geprüft werden, ob der Calciumwert sich im normalen Bereich befindet.

Dieses Beispiel mit der Schwangerschaft ist immens wichtig, da erhöhte Calciumwerte oftmals zu Fehlgeburten führen (vergleiche https://www.parathyroid.com/blog/high-calcium-during-pregnancy-hyperparathyroidism). Aus eigener Erfahrung kann der Erfinder durch die Konsultation von vielen verschiedenen Ärzten feststellen, dass die Bewertung von Calciumwerten in der Schwangerschaft als Risikofaktor nicht erkannt oder unterschätzt wird, da Gynäkologen davon ausgingen, dass Calciumwerte während der Schwangerschaft schwanken und Endokrinologen sich oftmals nicht mit den Auswirkungen auf die Schwangerschaft auskennen. Dies zeigt, dass die Erfindung eines automatisierten Apparates 180 ist notwendig ist um solche Fälle automatisiert und ohne menschliches Zutun aufzudecken. Die Erfindung 180 macht es somit möglich, dass auch Laien schnell Hinweise 190 und Bewertungen auf Krankheiten erhalten, was überlebenswichtig ist. Selbstverständlich lässt sich selbiges Verfahren auch mit Alter und Schilddrüsenwerten und weiteren Patientenmetadaten 100 durchführen, um Krankheiten besonders zielgenau bewerten zu können.

Die Ausgabe der Hinweise 190 kann besonders vorteilhaft im sogenannten Blutbild oder Laborbild erfolgen, aber es kann auch durch Hinweise 190 direkt in der Software bei der Darstellung auf einem Bildausgabegerät geschehen. Es ist dabei anzumerken, dass eine Ausgabe 190 natürlich auch direkt an den Patienten erfolgen kann, wenn z. B. dessen Telefonnummer oder dessen Email in den Patientennetadaten 100 vorhanden ist. Hierbei ist es besonders bei Handlungsempfehlungen 190 vorteilhaft, diese direkt dem Patienten zuzustellen. Zum Beispiel kann somit ein Patient per Kurznachricht oder Email für die Freigabe einer automatisierte Handlungsanweisung 190 benachrichtigt werden, die er dann durch eine Antwort oder einen Hyperlink bestätigen oder verweigern kann.

Durch diese automatisierten Handlungsanweisungen 190 wird eine Wechselwirkung mit dem Körper vermieden und relevante Laborwerte 110 stehen schneller, ohne die Notwendigkeit für mehrere Blutabnahmen oder Urinabgaben, zur Verfügung.

2.
In einer weiteren Ausführungsform können Laborwerte 110 besonders hilfreich in technischen Datenstrukturen 800 verarbeitet und empfangen werden. Solche Datenstrukturen 800 sind z. B. Maps, Bäume, CSV (comma separated values), Hashmaps, Json Daten, Datenbanktabellen und Key-Value Daten 800, die es erlauben Zuordnungen abzubilden.

Dadurch können Laborwerte 110 als Schlüssel 240 (Schlüssel 240 des Laborwertes kurz Laborwertschlüssel 110) definiert werden, der dann Werten 250 und einer physikalischen Einheit zugeordnet ist. Selbstverständlich können hier noch Erweiterungen durch weitere Felder, die ein Kalenderdatum oder andere Vermerke, wie z. B. Umrechnungsvorschriften oder der Verweis darauf, erfolgen.

Ein Schlüssel 240 ist z.B. eine Spezifikation des konkreten Laborwertes 110, was z. B. durch den Namen erfolgen kann, wie z. B. "TSH". Ein Wert 250 ist z. B. einem Schlüssel 240 zugeordneter Wert 250, wie z. B. 0,5. Werte 250 können dabei als Zahlen, aber auch als komplette Datenstrukturen, wie Strings, Arrays, Dateien, Listen, Tabellen, Bäume oder Graphen auftreten. Zuletzt kann einem Wert 250 eine oder mehrere physikalische Einheiten zugeordnet werden. Beispielsweise können physikalische Kenngrößen wie z. B.: mmol/L oder mg/dL in einer solchen Datenstruktur 800 als Feld abgelegt werden. Dies ist besonders vorteilhaft, da Laborwerte 110 oftmals in unterschiedlichen Einheiten vorliegen und somit die Erfindung 180 verschiedene Einheiten umrechnen und verarbeiten kann. Daher macht es eine Strukturierung mit Einheitenumrechnungen zwischen verschiedenen physikalischen Kenngrößen möglich.

Dies ist aber nicht der einzige technische Grund, um besonders vorteilhaft Datenstrukturen 800 einzusetzen. Oftmals ist es unklar, welche genauen Werte 250 Laborbefunde 110 beinhalten. Durch eine schlüsselbasierte Datenstruktur 800 ist die Erfindung 180 universell und besonders dynamisch einsetzbar, da somit Schlüssel 240 zur Steuerung der internen Verarbeitung genutzt werden können. Beispielsweise können Schlüssel 240 dazu genutzt werden zugehörige Referenzbereiche 130 in der Speichereinheit 120 aufdecken, die darin den gleichen Schlüsseln 240 codiert sind.

Aus diesem Grund kann die Erfindung 180 natürlich noch über eine Schlüssel-Zuordnungsfähigkeit 240 verfügen, da oftmals Laborwerte 110 unterschiedlich bezeichnet werden. Dies bedeutet, dass verschiedene Schlüssel 240 durch Regeln 90 auf einen internen Hauptschlüssel 240 abgebildet werden. Zum Beispiel kann ein Laborwert 110 in unterschiedlichen Sprachen vorliegen. Eine Schlüssel-Zuordnungsfähigkeit 240 kann daher z. B. : "Freies T4" wird "Free T4" zugeordnet; "F T4" wird "Free T4"zugeordnet usw., sein. Dies ermöglicht es besonders vorteilhaft mit unterschiedlichen Bezeichnern zu arbeiten. 3.

Besonders vorteilhaft können die Referenzwerte 130/240/250 in der Speichereinheit 120 der Erfindung 180 als Datenstruktur 800 verfügbar sein, die als Schlüssel 240 des Referenzwertes (kurz: Referenzwertschlüssel) mit assoziiertem Referenzwert 130/250 definiert ist. Hierbei kann die physikalische Einheit eines Referenzwertes 130 natürlich auch im Referenzwert 130/250 selbst oder als weiteres Feld vorhanden sein. Ein Referenzwertschlüssel 240 ist demnach ein Bezeichner für den konkreten Wertetyp, der direkt einem Laborwertschlüssel 240 oder einer generellen Zuordnungsfähigkeit mit einem solchen übereinstimmt. Dies bedeutet, dass durch den Laborwertschlüssel 240 besonders vorteilhaft passende Referenzwerte 250 aus der Speichereinheit 120 aufgedeckt werden können.

Ein Referenzwert 130/250 kann dabei ein von-bis Bereich sein. Ein Referenzwert 130/250 kann aber auch als Standardabweichung als Wert+-X darstellen. Weiterhin sind auch kompliziertere Referenzwerte 130 wie ganze Datenstrukturen 800, wie Histogramme und andere Verteilungen als Referenzwerte 130 möglich.

Besonders vorteilhaft wird als ein Laborwert 110 und dessen Schlüssel 240 (Laborwertschlüssel) verwendet, um passende Referenzwertschlüssel 130/240 in der Speichereinheit 120 aufzudecken und somit die passenden Referenzbereiche 130 zu bestimmen. Dann wird im nächsten Schritt geprüft, ob der Laborwert 110 sich inmitten des Referenzbereichs 130 befindet. Beispielsweise kann dies wie folgt passieren: "unterer Referenzbereich" < "Laborwert" < "oberer Referenzbereich". Selbstverständlich werden zuvor noch die Einheiten des Referenzwertes 130 und des Laborwerts 110 geprüft und gegebenenfalls werden die Einheiten umgerechnet.

Gemeinhin können Referenzwerte 130 auch mit Ausgaben 190 wie Hinweisen, Handlungsempfehlungen, (maschinenlesbare/durchführbare) Handlungsanweisungen, Diagnoseempfehlungen, Risiken und Anweisungen für die Unter-oder Überschreitung, versehen sein. Hierbei können auch Regeln 90 hinterlegt sein, bei welchen Abweichungen welche Handlungsempfehlungen, Handlungsanweisungen 190 oder ähnliches ausgegeben werden.

Eine Handlungsanweisung 190 kann dabei sein, dass automatisiert noch ein weiterer Laborwert 110 bestimmt wird. Beispielsweise kann bei einem hohen Calciumwert 110 die Handlungsanweisung sein automatisiert das Parathormon (PTH) 110 zu messen.

Weiterhin können Referenzwerte zusätzlich als eine oder mehrere Gruppen vorhanden abgelegt sein, sodass überprüft wird, inwiefern verschiedene Werte gleichzeitig über- oder unterschritten werden. Zum Beispiel:
PTH: >67 ng/1.; Calcium: > 2.54 mmol/l; Vitamin D: < ng/ml; -> Hinweis: Vermutliche Erkrankung an Hyperparathyreoidismus.

Wie somit zu sehen ist, können durch die Datenstrukturen 800 einfach Referenzwerte 130 oder auch Referenzwertgruppen 130 überprüft werden. Besonders vorteilhaft ist diese Datenstruktur 800 einzusetzen, indem die Referenzwerte 130 mit Rahmenbedingungen 200 von Patientenmetadaten 100 verknüpft oder mit Regeln 90 zur Auswertung verbunden werden.

4.
Besonders vorteilhaft können auch Regeln 90 alleine oder in Kombination mit Referenzbereichen 130 verwendet werden.

Beispielsweise wird der passende Referenzwert 130 aufgrund der Patientenmetadaten 100 für die richtige Altersgruppe aufgrund der Patientenmetadaten 100 bestimmt. Es ist somit ein z. B. altersgerechter TSH-Referenzbereich 130 verfügbar.

Regeln 90 können zusätzlich zur normalen Auswahl von einem Patientenmetadatum 100 dazu verwendet werden, um eine Multi-Faktor-Auswahl von Referenzbereichen 130 durchzuführen. Beispielsweise können Free T4, Free T3 und TSH als Referenzwerte 130 von Geschlecht und zugleich Alter als Rahmenbedingung 200 abhängig sein. Daher sind Referenzwerte 130 hierfür in der Speichereinheit 120 mit den Alter und Geschlecht als Rahmenbedingungen 200 verknüpft. Regeln 90 ermöglichen es hier zielgenau Referenzwerte 130 zu bestimmen, die besonders vorteilhaft zu Schlüsseln 240 und Werten 250 in den Patientenmetadaten 100 passen.

Es kann zudem in den Patientenmetadaten 100, vermerkt sein, dass der Patient sportlich ist (z. B. als: Fitness=Schlüssel 240; Wert 250=sportlich von Patientenmetadaten 100). Bei Sportlern verhält sich der TSH Laborwert 110 normalerweise etwas anders. Beispielsweise kann der vom Laborwert TSH 110, aufgrund von Sportlichkeit z. B. 0,5-1,0 mIU/mL subtrahiert oder addiert werden, um einen jeweils passenden Vergleich zu einem sportlichen oder unsportlichen Menschen zu haben. (Vergleiche hierzu: TSH Werte Bansal A, Kaushik A, Singh C M, Sharma V, Singh H. The effect of regular physical exercise on the thyroid function of treated hypothyroid patients: An interventional study at a tertiary care center in Bastar region of India . Arch Med Health Sci [serial online] 2015 [cited 2022 May 7];3:244-6. https://www.amhsjournal.org/text.asp?2015/3/2/244/171913 ).

Regeln 90 können einen solchen Vergleich zwischen sportlich und unsportlich und die Auswirkung auf physikalische Größen einfach ermöglichen. Beispielsweise könnte der TSH Laborwert 110 im einfachsten Fall angepasst werden. Alternativ und semantisch stimmiger ist jedoch die Ausführung der Erfindung 180, die eine oder mehrere dynamische und besonders flexible Anpassungen des Referenzbereichs 130 durch eine oder mehrere Regeln 90 ermöglicht. Beispielsweise können die oberen und/oder unteren Grenzwerte um 0,5 eines Referenzwertbereichs 130 verschoben werden, wenn die Laborwerte 110 von einem Sportler stammen.

Das Anpassen der Referenzbereiche 130 hat besondere Vorteile, da somit mehrere Regeln 90 aufeinander aufbauend kombiniert werden können. Beispielsweise ist der TSH Laborwert 110 bei Frauen oft niedriger als bei Männern (Suzuki, Satoru et al. "Gender-specific regulation of response to thyroid hormone in aging." Thyroid research vol. 5,1 1. 26 Jan. 2012, doi:10.1186/1756-6614-5-1). Durch die unterschiedlichen physikalischen Kenngrößen (Referenzbereiche 130) bei Frauen und Männern könnten diese Unterschiede in Regeln 90 in der Speichereinheit 120 codiert sein, wodurch die Referenzbereichsgrenzen 130 der Sportlichkeit (0,5 mIU/mL) hinzu z. B. um +-0,4 mIU/ml bei Frauen zusätzlich angepasst werden könnten, was z. B. zu einer Gesamtanpassung eines Referenzwertbereichs 130 um 0,9 mIU/ml führt.

Ein weiteres Beispiel für eine solche Regel 90 ist, dass das Schwangerschaftshormon Beta-
HCG eine Strukturgleichheit mit dem TSH hat und somit im 01. Schwangerschaftsdrittel ausgeschüttet wird, wodurch der TSH-Spiegel der Schwangeren sinken (https://www.endo-bochum.de/endokrinologie/tsh-spiegelthyreoida-stimulierendes-hormon/ ). Dieser Umstand kann durch die Erfindung 180 nun besonders vorteilhaft durch eine Regel 90 Referenzbereichsanpassung 130 bei Patientenmetadaten 100, die eine Schwangerschaft im ersten Trimester beinhalten, beachtet werden.

5.
Regeln 90 können auch besonders bevorzugt verwendet werden, um eine Vielzahl von Zusammenhängen aufzudecken, wie zum Beispiel wenn mehrere Referenzbereiche 130 gleichzeitig über- oder unterschritten werden oder wie sich verschiedene Laborwerte 110 in Kombination zueinander verhalten.

Die Kombination aus Patientenmetadaten 100, Regeln 90 und Rahmenbedingungen 200, wie z. B. Alter (Rahmenbedingungen 200 und Patientenmetadaten 100 Schlüssel "Alter" (240) und Werte "konkretes Alter"(250)), macht es somit besonders vorteilhaft möglichst zielgenau Bewertungen vorzunehmen und auf die individuellen Patientenumstände einzugehen, um dann eine Mehrzahl von Referenzabweichungen 130 in ihrer Gemeinsamkeit zu beurteilen.

Selbiges kann nun auf mehrere andere Laborwerte 110 angewandt werden. Beispielsweise kann somit auch ein Vitamin D Laborwert 130 aufgrund der Patientenmetadaten 100 eines Wohnortes oder eines Berufes eines Patienten beurteilt werden. Beispielsweise ist ein Gärtner oder ein Einwohner eines tropischen Landes viel mehr der Sonne ausgesetzt oder jemand mit dunkler Hautfarbe produziert weniger Vitamin D wie ein hellhäutiger Hauttyp in Nordeuropa.

Selbstverständlich können Regeln 90 dazu verwendet werden, um solche Bewertungen zu kombinieren oder Bewertungen aufgrund mehrerer Patientenmetadaten 100 und mehrerer Laborwerten 110 vorzunehmen. D.h. Regeln 90 werden dazu verwendet eine Mehrzahl von Referenzwertabweichungen 130 oder eine Mehrzahl von Laborwerten 110 und Patientenmetadaten 100 in ihrer Gesamtheit zu beurteilen. Ein Beispiel: Ein hoher Calcium-Laborwert 110, ein niedriger Vitamin D Laborwert 110 und ein Lebensmittelpunkt in einem tropischen Land (Lebensmittelpunkt= Schlüssel 240 und Wert 250 von Patientenmetadaten 100 und Rahmenbedingungen 200) im Outdoorbereich führen zu der Bewertung: Verdacht auf Hyperparathyreoidismus (Ausgabe 190).

6.
In einer weiteren Ausführung der Erfindung 180 wird auch eine besonders vorteilhafte Datenstruktur 800 für Patientenmetadaten 100(120) und die Rahmenbedingungen 200 verwendet. Hierbei werden diese in einem Schlüssel(vereinfacht auch Metadatenschlüssel) - Wert 240/250 (vereinfacht auch Metadatenwert) Schema und gegebenenfalls weiteren Werte 250 strukturiert.

Ein Wert 250 kann dabei auch ein Wertebereich 250 sein und hierbei kann die Erfindung 180 über die Fähigkeit verfügen einzelnen Werten 250 einem Wertebereich 250 zuzuordnen. Beispielsweise ein Metadatenwert 250 (310), konkretes Alter (310), von Metadatenschlüssel 240 Alter, kann einer Altersgruppe 250 zugeordnet sein. Ein Beispiel: "Alter"(240): "25"(250) zugeordnet "Altersgruppe"(240): "25-29"(250).

Beispielsweise kann dies in den Patientenmetadaten 100 wie folgt aussehen:
- Alter(240): 29 (250)
- Geschlecht(240): weiblich (250)
- Diagnosen(240): Hyperthyreoidismus(250), Long Covid (250)
- Aktivitätslevel(240): Sportlich(250)

Ebenfalls sind Rahmenbedingungen 200 in der Speichereinheit 120 ähnlich definiert. z. B.
- Altersgruppe(240): 25-30: Referenzwerte(250)
- Aktivitätslevel(240): Sportlich: TSH +0,5 (90)
- Aktivitätslevel(240): Normal: (90)

Durch diese ähnlichen Datenstrukturen 800 von Rahmenbedingungen 200 und Patientenmetadaten 100 können nun, durch einen Vergleich von Schlüsseln 240 und Werten 250 der Patientenmetadaten 100 und Rahmenbedingungen 200, daraus Referenzwerte 130 durch Regeln 90 abgeleitet werden, die zu den Patientenmetadaten 100 passen.

Beispielsweise kann durch einen Altersbereich 250 in den Patientenmetadaten 100 eine Sammlung von Referenzwerten 130 für die verschiedenen Laborwerte 110 in der Speichereinheit 120 aufgedeckt werden. Durch zumindest eine Regel 90 kann dann ein Referenzbereich 130 um X physikalische Einheiten erhöht oder vermindert werden oder die Grenzwerte der Referenzwertbereiche 130 werden durch eine oder mehrere Regeln 90 nach oben oder unten angepasst. Nachfolgend kann dann der Vergleich der Laborwerts 110 mit den angepassten Referenzwerten 130 durchgeführt werden.

7.
In einer weiteren Ausführung kann zumindest eine Bewertung, durch eine automatisiert durchgeführte, nachführende Ermittlung 80 (auch Nachjustierungsanalysen oder kurz Nachführung genannt) zumindest eines weiteren Laborwerts 110, besonders vorteilhaft verbessert werden. Eine Verbesserung der vorhergehenden Ausgabe 190 ist hierbei zumindest eine Bestätigung, Widerruf oder Veränderung der Wahrscheinlichkeit der Korrektheit der vorhergehenden Bewertung 190.

Dies ist besonders hervorzuheben, da somit Patienten das Leid erspart werden kann mehrmals Körperflüssigkeiten abzugeben. Beispielsweise erzeugt eine Bewertung (auch Ausgabe genannt) 190 eine Vermutung und Handlungsanweisung und daraufhin wird direkt ein weiterer Laborwert 110 in der gleichen oder anderen Flüssigkeitsprobe (z. B. Blut und Urin), die schon zuvor abgegeben wurde, gemessen. Dies ist besonders vorteilhaft, um besonders ressourcenschonend Ausgaben 190 durchzuführen. Realisiert werden kann dies beispielsweise durch mit Referenzwerten 130 verknüpfte Anweisungen bei der Laboranalyse.

Ein Beispiel kann folgendes sein. Eine Bewertung /Ausgabe 190 zu einer Blutprobe führt zu dem Hinweis eines vermutlichen Schilddrüsenleidens durch einen TSH Wert, der von den Referenzwerten 130 abweicht. Automatisiert durch eine Handlungsanweisung 190 wird dann ein Test von Freiem T3 und T4 vorgenommen (erste Nachjustierung 80). Sofern diese Laborwerte 110 /130 im Normalbereich 130 sind und somit kein direktes Schilddrüsenleiden aufgedeckt (Laborwerte 110 im Referenzbereich 130) ist, kann fortfolgend automatisiert (zweite Nachjustierung 80) ein Antikörpertest von TPO Antikörpern durchgeführt werden, der hier durch die Laborwerte 110 ermittelt und diese mit dem Referenzbereich 130 vergleicht. Sofern diese erhöht (z. B. Anti-TPO oder auch TPO-AK ("Thyreoperoxidase-Antikörper") > 150 U/ml) sind, kann die Ausgabe 190 mit einem Hinweis auf die Krankheit Hashimoto erfolgen.

Durch die Erfindung 180 werden somit automatisiert durch Handlungsanweisung 190 und Nachjustierung 80 sukzessive relevante Laborwerte 110 schnell und effizient ermittelt. Dies entlastet medizinisches Personal und Patienten, da somit ohne menschliche Wechselwirkung besonders schnell passende Nachführungen 80 möglich werden.

8.
In einem weiteren Aspekt der Erfindung 180 können besonders vorteilhaft Geodaten 999 in den Patientenmetadaten 100 verwendet werden. Geodaten 999 können zum Beispiel GPS Daten oder Adressen sein.

Geodaten 999 (auch Geoinformation, Standortinformation, Standortverlauf, Standorthistorie und ähnlich genannt) können dabei z. B. i. Standort 999 der Entnahme von Blut und Urin, ii. Standorte 999 einer Untersuchung entsprechen oder iii. vergangene Standorte 999 des Patienten entsprechen. Optimalerweise enthalten Standorte 999 eine Verweildauer.

Dies ist besonders vorteilhaft, um geospezifische Rahmenbedingungen 200 und zugehörige Referenzbereiche 130 oder anzuwendende Regeln 90 aufgrund von Geodaten 999 in den Rahmenbedingungen 200 der Speichereinheit 120 aufzudecken.

Dies ermöglicht es besonders effizient Referenzbereiche 130 zu verwenden, die passend sind und noch vielmehr Handlungsempfehlungen 190 oder auch automatisierte Handlungsanweisungen 190 aufgrund von Geodaten 999 anzupassen. Somit können z. B. Handlungsanweisungen 190 direkt berücksichtigen welche Krankheiten in einer Georegion 999 häufig sind und daraus konkrete Ermittlungen von weiteren Laborwerten 110 spezifizieren, die in einer Region besonders vorteilhaft für Referenzwertvergleiche 130 sind.

9.
Standorte 999 eines Patienten können dabei z. B. durch ein Smartphone aufgezeichnete vergangene Geopositionen 999 oder durch einen Standortverlauf 999 aus einer Cloud über ein Computernetzwerk 220 übertragen werden. Weiterhin ist es natürlich möglich, dass GPS Daten manuell aufgrund einer Adresse ermittelt werden und dann zu den Patientenmetadaten 100 hinzugefügt werden.

Selbstverständlich können GPS Daten auch in ungenauere Formen wie Bereiche, Stadtviertel, Regionen oder Länder transformiert werden, die dann als Patientenmetadaten 100 verwendet werden.

GPS Daten oder Daten die daraus gewonnen wurden (wie Aufenthaltsbereich, Regionen etc. ) werden besonders vorteilhaft verwendet, um Laborwerte 110 zu bewerten. Beispielsweise können somit Vitamin D Laborwerte 110 aufgrund von der Sonneneinstrahlung an einer Geoposition 999 oder einem Umkreis bewertet werden. Sofern ein niedriger Vitamin D Laborwert 110 in einem saisonalen Land existiert und es dort Winter ist, fällt die Bewertung/Ausgabe 190 eines niedrigen Vitamin D Wertes anders aus als bei einem tropischen Standortwert 999. Ein niedriger Vitamin D Laborwert 110 in einem tropischen Land kann z. B. zu einer Empfehlung 190 zu weiteren Untersuchungen für ein Stoffwechselproblem führen.

Selbstverständlich können auch weitere Faktoren aus den Patientenmetadaten 100 miteinbezogen werden, wie z. B. ob ein Patient in einem tropischen Land in einem Bergwerk arbeitet, womit die Bewertung anders ausfallen muss als bei einem Gärtner.

Besonders vorteilhaft kann ein Standortverlauf 999 sein. Man stelle sich vor, dass man bei einer Urlaubsreise in den Tropen mit dem Dengue Fieber oder Malaria infiziert wird. Nach einer Inkubationszeit treten im Heimatland, wie z. B. Deutschland, Symptome auf und Laborwerte 110 werden bestimmt. Jedoch werden nur deutsche Standardwerte beauftragt, wodurch kein Dengue Laborwert 110 verfügbar ist. Im Normalfall fällt bei Dengue Fieber die Thrombozytenzählung. Ein Verdacht auf Dengue Fieber liegt in Deutschland nicht nahe, da die Krankheit nicht heimisch ist. Durch die Erfindung 180 wird nun aber der Standortverlauf 999 aus dem Smartphone des Patienten (oder alternativ durch manuelle Eingabe der letzten bereisten Länder oder Regionen) mit zur Bewertung einbezogen. Durch Regeln 90 (z. B. niedrige Thrombozytenzählung und Standorte 999 in tropischen Ländern: Handlungsanweisung 190 Dengue Fieber Laborwert 110 ermitteln) kann eine Handlungsempfehlung oder Handlungsanweisung 190 einen Dengue Fieber Laborwert 110 zu ermitteln ausgegeben oder direkt durchgeführt werden. Additiv kann eine Handlungsempfehlung zur Tötung von Moskitos (z. B. durch Begasung von Bereichen die am Wasser liegen) mitsamt der letzten Aufenthaltsorte des Patienten aus dessen Smartphonedaten ausgegeben werden, damit sich die Krankheit nicht weiter ausbreitet.

10.
Oftmals haben Schwangere Angst ihr Embryo/Fötus/Kind zu verlieren. Daher kann die Erfindung 180 in einem weiteren Aspekt besonders vorteilhaft als Patientenmetadatum 100 dazu verwendet werden, um zumindest ein Risiko 190 einer Schwangerschaft aufzudecken.

Beispielsweise wird somit ein Patientenmetadatum 100 wie lange die Patienten schwanger sind dazu verwendet, um Laborwerte 110 auf Risiken zu bewerten bzw. Ausgaben 190 hiervon zu erzeugen. Unter Anderem führen z. B. leicht erhöhte Calcium Laborwerte 110 schon zu einem sehr hohen Schwangerschaftsverlustrisiko. Neugeborene tragen zusätzlich das Risiko mit einer Hypokalzämie geboren zu werden. Durch das Verwenden der Information, dass ein Patient schwanger ist, kann somit der Referenzbereich 130 von Calcium Laborwerten 110 in der Schwangerschaft und Referenzbereich 130 mit Risiken 190 verglichen werden. Fortfolgend können dann die Risiken der Schwangerschaft direkt bewertet und ausgegeben werden.

Besonders vorteilhaft kann eine Datenbank aus Referenzwerten 130, Regeln 90 und Risiken verwendet werden (Beispiel für Datenstrukturen 800). Optimalerweise werden alle potentiellen Risiko-Laborwerte 190/110 aus einer Blutprobe ermittelt. Fortfolgend werden diese Laborwerte 110 beurteilt, indem diese mit Referenzwerten 130 in der Schwangerschaft verglichen werden, wobei die Referenzwerte 130 zuvor durch die persönlichen Patientenmetadaten 100 der Schwangeren angepasst werden. Somit können individuell Abweichungen zu Referenzwerten 130 in der Schwangerschaft ermittelt und die speziellen Referenzwertanpassungen 130 als Hinweise 190 ausgegeben werden. Zusätzlich können Abweichungen noch als Risiken 190 mit ausgegeben werden.

11.
Oftmals haben Paare einen unerfüllten Schwangerschaftswunsch. Daher kann eine weitere Ausführung der Erfindung 180 besonders vorteilhaft Patientenmetadaten 100 verwenden, um Laborwerte 110 auf Fruchtbarkeitseinschränkungen zu beurteilen.

Dies funktioniert z. B. wie folgt: Patientendaten 100, wie z. B. Body Mass Index, Größe, Gewicht, Alter, Regelmäßigkeit der Periode, Tag der letzten Periode, Regelmäßigkeit von sportlichen Aktivitäten und /der Diagnosen wie z. B. Diabetes oder Polyzystisches Ovarialsyndrom (PCO) werden verwendet, um Laborwerte 110 im Hinblick auf Fruchtbarkeitseinschränkungen zu bewerten. Beispielsweise kann die Beurteilung wie folgt beeinflusst werden:
- Eine Beurteilung auf eine Fruchtbarkeitseinschränkung aufgrund von Schilddrüsenwerten (Thyreoideastimulierendes Hormon (TSH)) kann durch eine Information, dass der Patient am Polyzystischen Ovarialsyndrom erkrankt ist, beeinflusst werden, sodass Abweichungen von der Norm verstärkt als Fruchtbarkeitseinschränkung gewertet werden. Beispiele unter der Anwendung von Beurteilungsregeln 90 (ohne Einheiten zur Vereinfachung):
- Ein Patient ist 30 Jahre alt, führt regelmäßig Sport durch und hat einen TSH Laborwert 110 von 3,7. Aufgrund des Alters ist der TSH Laborwert 110 als normal zu bewerten. Jedoch sollte er aufgrund des regelmäßigen Sports unter 3 sein. Daher wird hier eine potentielle Fruchtbarkeitseinschränkung beurteilt.
- Ein Patient ist 30 Jahre alt und hat einen Calcium Laborwert 110 von 2,6, der eigentlich noch im Referenzbereich 130 (bis 2,65) liegt. Aufgrund des Schwangerschaftswunsches ist der Laborwert 110 jedoch als potentielle Fruchtbarkeitseinschränkung zu werten. Weiterhin wird die Fruchbarkeitseinschränkung noch durch den speziellen Referenzbereich 130 des Alters verstärkt (Calcium mit 30 Jahren sollte unter dem Wert 250 von 2.6 sein). Somit kann eine potentiell starke Fruchtbarkeitseinschränkung durch hohe Calcium Laborwerte 110 ausgegeben werden. Weiterhin kann ein weiterer Hinweis 190 ausgegeben werden, woher die Fruchtbarkeitseinschränkung kommen könnte.

Insgesamt gesehen werden also Regeln 90 und Referenzbereiche 130 aufgrund von Patientenmetadaten 100 verwendet, um besonders vorteilhaft Fruchtbarkeitseinschränkungen aufzudecken.

12.
Ein weiteres Beispiel, wie die Erfindung 180 besonders effizient Hinweise durch Nachjustierungsanalysen 80 verbessern kann, ist der folgende Einsatz:
Durch erhöhte Calcium Laborwerte 110, bezogen auf Patientenmetadaten 100 und Referenzwerten 130, wird ein Hinweis 190 für eine potentielle Diagnose auf Hyperparathyreoidismus erzeugt. Weiterhin wird die Handlungsanweisung 190 erzeugt, automatisiert einen Laborwert 110 von Vitamin D und optimalerweise von Urin, den Calciumwert zu bestimmen.

Danach wird eine Bewertung aufgrund von Regeln 90 durchgeführt. Sofern der Vitamin D Laborwert 110 niedrig und der Calcium Laborwert 110 im Blut und Urin hoch ist, wird die Handlungsanweisung 190 durchgeführt, automatisiert ein Parathormon Laborwert 110 (PTH) zu ermitteln (Nachjustierung 80). Selbstverständlich könnte der PTH Test auch gleich sofort bei einem hohen Calcium Laborwert 110 durchgeführt werden. Jedoch sollte die mindestens zweistufige Nachbesserung (80) an diesem Beispiel erklärt werden.

Sofern der PTH Laborwert 110 zu den anderen zuvor beschriebenen Laborwerten 110 (Urin Calcium, Calcium Blut, Vitamin D) am oberen Ende des Referenzwertes liegt, kann eine Bewertung/Ausgabe 190 eine sehr hohe Wahrscheinlichkeit von Hyperparathyreoidismus indizieren und die Diagnose oder einen Diagnosehinweis ausgegeben werden. Besonders vorteilhaft können noch weitere Hinweise 190 ausgeben werden, wie z. B. dass mit absolut hoher Wahrscheinlichkeit sicher ein Adenom an einer Nebenschilddrüse vorhanden ist und zumindest eine Nebenschilddrüse operativ entfernt werden sollte. Sofern die Patientenmetadaten 100 eine Schwangerschaft beinhalten, kann zudem der Hinweis/Risiko/Warnung ausgegeben 190 werden, dass eine sofortige Operation erfolgen sollte, da der Fötus in Gefahr einer Totgeburt ist.

Dieses Beispiel zeigt sehr deutlich die besonderen Vorteile dieser Ausführungsform, da somit eine komplexe Diagnose und Hinweise 190 nicht nur von absoluten Experten durchgeführt werden können. Erst durch das Bewerten durch Patientenmetadaten 100 (wie z. B. Alter) können Abweichungen erkannt und seltene Krankheiten erkannt werden. Die besondere Dynamik zeigt dabei noch, dass automatisiert und mehrstufig zugehörige Laborwerte 110 ermittelt werden können. Zusätzlich ermöglicht dies, ohne menschliche Fehlerkomponenten, relevante Laborwerte 110 aufzudecken und hilft dann durch die Ausgabe von Hinweisen 190, dass Ärzte und Patienten wesentlich schneller als in einem manuellen Verfahren mit mehreren Untersuchungen, Entscheidungen treffen können.

13.
In einem weiteren Aspekt der Erfindung 180 kann die Erfindung 180 besonders vorteilhaft Daten aus vorherigen Diagnosen und Behandlungen nutzbar machen.

Hierzu verfügt die Erfindung 180 über eine Speichereinheit 120/500, in der Patientenmetadaten 100, Behandlungen und Behandlungserfolge 510 gespeichert werden können. Hierbei ist anzumerken, dass Behandlungserfolg 500 selbstverständlich auch Misserfolge oder mehr Details zu der Behandlung und den Fortschritten enthalten kann. Besonders bevorzugt sind die Daten auch pseudonymisiert oder anonymisiert gespeichert. Die Speicherung in der Speichereinheit 120/500 kann dabei in einer Datenbank oder auch in einer Cloud erfolgen, an die Daten 510 geliefert werden.

Die Erfindung 180 vergleicht die Datensätze der Patienten auf Ähnlichkeit. Dabei können z. B. Patientenmetadaten 100 und Laborwerte 110 verglichen werden, um besonders ähnliche "Patienten-Rahmenbedingungen" (der einfacheren Lesbarkeit oftmals auch nur kurz Patienten genannt) aufzudecken.

Aufgrund der Ähnlichkeit können somit ähnliche Datensätze von Patienten verglichen und aufgedeckt werden. Deren Behandlungserfolg und Behandlungsmethode 510 kann nach einem Vergleich 530 ausgegeben werden. Dies ist besonders vorteilhaft, weil damit potentiell Millionen von Datensätzen durch die Ähnlichkeitsvergleiche 530 schnell und effizient gefiltert werden und die am meisten relevanten Fälle nun von Experten oder weiteren Systemen verarbeitet werden können.

Besonders vorteilhaft können die ähnlichen Werte der anderen Patienten mit ausgegeben und die Abweichungen dazu sichtbar gemacht werden. Diese Ausgabe 190/520 kann z. B. durch eine tabellarische Anzeige oder anderem aus dem Stand der Technik bekannte Anzeigen oder Ausgabe-Techniken geschehen.

14.
Die vorliegende Erfindung 180 betrifft ferner ein Verfahren für das Erstellen von Ausgaben 190 in der Form von Hinweisen, Risiken, Handlungsempfehlungen, Handlungsanweisungen und Ursachenhinweisen aufgrund von Dokumenten, die Laborwerte 110 und Patientenmetadaten 100 beinhalten, wobei das Verfahren zumindest die folgenden Schritte aufweist:
a. Speichern zumindest eines Referenzwertes 130 bestehend aus Schlüssel 240 und Wertbereich 250 eines Laborwertes 110 mitsamt zumindest einer Zuordnung zu zumindest einer Rahmenbedingung 200;
b. Übertragung zumindest eines Patientenmetadatums 100 mitsamt zumindest einem Laborwert 110, bestehend aus Schlüssel 240 und Wert 250, über zumindest ein Computernetzwerk 220;
c. Aufdecken von zumindest einem gespeicherten Referenzwert 130 aus a. unter dem Vergleich von
   I. zumindest einem Patientenmetadatum 100 aus b. mit zumindest einer Rahmenbedingung 200; und
   II. zumindest einem Laborwert 110 Schlüssel 240 aus b. mit zumindest einem Referenzwert Schlüssel 240 aus a.;
d. Vergleich von zumindest einem Laborwert 110 Wert 250 aus b. mit zumindest einem in c. bestimmten Referenzwert 130;
e. Zumindest eine Ausgabe 190 aufgrund zumindest eines Ergebnisses eines Vergleiches aus d.

Damit wird die Patienteninformation/Patientenmetadatum 100, wie z.B. Schwangerschaft, verwendet um passende Rahmenbedingungen 200 beinhaltend Referenzwerte 130 für diese aufzudecken. Der Schlüssel 240 des Laborwertes 130 (Schlüssel 240 des Laborwertes, wie z.B. eine Spezifikation des konkreten Laborwertes 110, was z. B. durch den Namen erfolgen kann, wie z. B. "TSH") selektiert somit den passenden Schlüssel 240 der Referenzwerte 130, damit dann der Laborwert 130 Wert 250 mit dem passenden Referenzbereich 130 Wertebereich verglichen werden kann.

15.
Zuletzt stellt die vorliegende Erfindung 180 ein Computerprogramm bereit, das Instruktionen aufweist, um jedes in der Anmeldung beschriebene Verfahren auszuführen und/oder um eine der beschriebenen Anordnungen oder Systeme durch ein Computerprogramm einzeln oder in Kombination zu repräsentieren.

### 4. Kurzbeschreibung der Zeichnungen

Fig. 1: Eine Ausführungsform der Erfindung 180, um Laborwerte 110 anhand von Patientenmetadaten 100 angepasste Referenzbereiche 130 durch Regeln 90 zu bewerten und Ausgaben 190 auszugeben. Optional sind Nachjustierungsanalysen 80 gezeigt, die automatisiert ausgegebene Handlungsanweisungen 190 verarbeiten.
Fig. 2: Eine Ausführungsform der Erfindung 180, die direkt in einem Diagnosegerät oder Diagnoseprogramm 600 eingesetzt wird.
Fig. 3: Ein exemplarischer Datenstruktur-Inhalt 800 einer Speichereinheit 120 der Erfindung 180.
Fig. 4: Eine Datenstruktur 800, wie Sie für Referenzlaborwerte 110/130, Rahmenbedingungen 200, Patientenmetadaten 100 und Ausgaben 190 in der Erfindung 180 besonders vorteilhaft in derer Speichereinheit 120 verwendet werden kann.
Fig.5 Eine Ausführungsform der Erfindung 180 mit einer speziellen Speichereinheit 500/120 in der konkrete Behandlungsdaten von Patienten gespeichert werden, die über eine Vergleichseinheit 530 verfügt, die es ermöglicht Patientenmetadaten 100 durch verschiedene Methoden zu vergleichen.

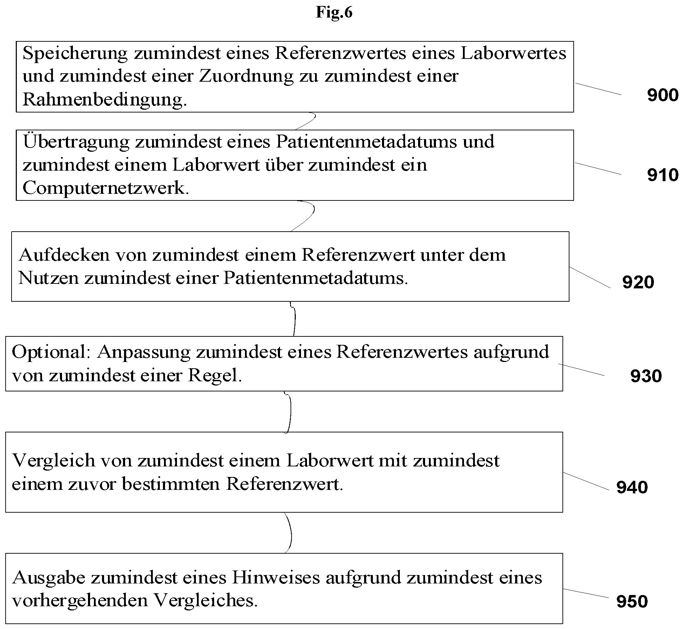

Fig. 6. Eine Ausführungsform der Erfindung 180 als Verfahren

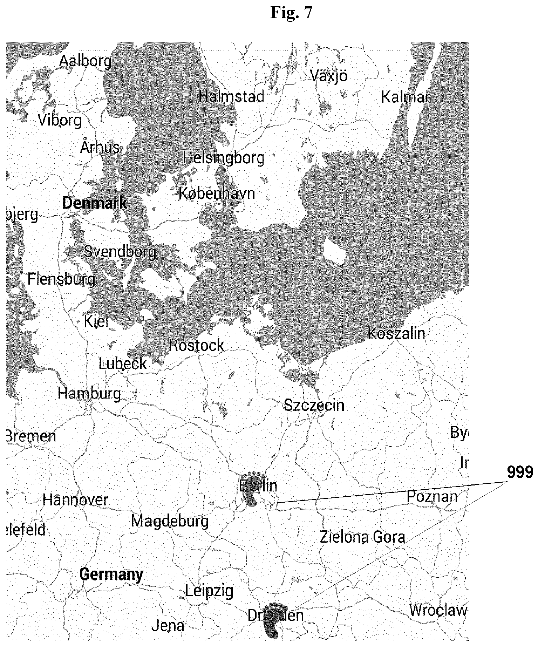

Fig. 7. Ein Beispiel für Geopositionen 999 und eine Standorthistorie 999, wie sie besonders bevorzugt in der Erfindung 180 genutzt werden können.

### 5. Detaillierte Beschreibung von bevorzugten Ausführungsformen

### Definitionen:

Gleiche Beschriftungen durch Zahlen (Referenzen) im Text und Figuren zeigen den gleichen Sachverhalt. Oftmals ist ein Sachverhalt aus verschiedenen Blickwinkeln oder in verschiedenen Granularitäten zu betrachten, weshalb ein Sachverhalt oftmals durch verschiedene Zahlen (Referenzen) bezeichnet wird. Eine Nennung einer Referenz stellt demnach keine Einschränkung dar, sondern vielmehr soll es dem Leser besonders einfach ermöglicht werden die Grafiken mit der oder den am besten verständlichen Sachverhalten in den Figuren zu verknüpfen, die im referenzierenden Text beschrieben werden.

Die Erfindung 180 kann natürlich auch direkt oder indirekt mit einem oder mehreren Systemen zusammen wirken. Beispielsweise kann diese Teil eines Systems sein oder ein System kann auch ein Teil von der Erfindung 180 sein. Das ermöglicht eine nahtlose Integration mit weiteren Systemen. Dadurch ergibt sich eine besonders vorteilhafte Verteilbarkeit, wie zum Beispiel in einem Computernetzwerk 220 über mehrere Server.

Ein Computernetzwerk 220 stellt dabei im Sinne dieser Erfindung 180 immer einen möglichen Plural von Netzwerken dar und ist nicht nur auf ein Netzwerk beschränkt. Vielmehr können verschiedene Computernetzwerke 220 als Gemeinsamkeit als Computernetzwerk 220 bezeichnet werden. Weiterhin können Computernetzwerke 220, die mit anderen Computernetzwerken 220 interagieren selbst als Computernetzwerk 220 bezeichnet werden. Aufgrunddessen ist in diesem Dokument der Ausdruck Computernetzwerke 220 und Computernetzwerk 220 als äquivalent anzusehen. Selbstverständlich stellen auch Mobilfunknetzwerke Computernetzwerke 220 dar. Generell ist der Ausdruck Netzwerk 220 in dieser Anmeldung mit dem Ausdruck Computernetzwerk 220 gleichzusetzen. Oftmals, wird jedoch die Nummer 130 bei dem Ausdruck Netzwerk oder Computernetzwerk 220 nicht gesetzt, weil sich nicht direkt auf einen wichtigen Sachverhalt der Figuren bezogen wird, um die Lesbarkeit zu erhöhen.

Da die Erfindung 180 insbesondere als datenverarbeitendes System/Verfahren/Anordnung konzipiert wurde, nutzt diese besonders vorteilhaft aus dem Stand der Technik bekannte Verfahren/Anordnungen/Systeme und Mechanismen aus, um bestmögliche Ausführungsresultate zu erzielen. Insbesondere können hierbei bekannte Systeme/Verfahren/Anordnungen aus dem Bereich Mikrochipgesteuerte Geräte, wie Computer oder Smartphones, verwendet werden. Aus diesem Grund bezieht sich die Erfindung 180 an einigen Stellen auf die Verarbeitung von Daten. Unter dem Term Verarbeitung sind daher im Sinne der Erfindung 180 z. B. das Nutzen der Daten in einem Computerprogramm, Berechnungen, Transformationen, Assoziation, Abfrage, Auswertung, die Ablage in einer oder mehreren Speichereinheiten oder ähnliche aus dem Stand der Technik bekannte datenverarbeitende Mechanismen und Verfahren zu verstehen.

In dieser Anmeldung und in den Ansprüchen wird das Word Dokument oder ein geospezifisches Dokument verwendet. Dokumente sind dabei nicht nur als Dokumente im klassischen Sinne zu verstehen. Vielmehr können Dokumente auch aktive Ressourcen sein, wie beispielsweise ein Webserver oder mehrere Webserver, eine Datenbank oder auch mehrere Datenbanken, von denen dann die Informationen geladen werden. Dabei bezieht sich der Begriff Dokument auch auf mehrere Systeme oder auch über Computernetzwerke 220 gekoppelte Systeme. Der Begriff Dokument ist demnach ein Sammelbegriff für alle möglichen Ausgaben einer Datenbasis oder auch eine Zusammenstellung von benutzererzeugten Daten. Ein Dokument kann z. B. dynamisch von einer Datenbasis generiert werden, weshalb eine Datenbasis im Rahmen der vorliegenden Erfindung 180 als Quelle oder Kombination von Quellen oder Quellsystemen anzusehen ist, von dem Daten angefordert, geschrieben und/oder geändert werden können. Ein Dokument ist demnach eine logische Einheit, eine Partition, Komponente und/oder Unterteilung und trifft daher auch auf Teile von Dokumenten zu. Ausführungsformen der vorliegenden Erfindung 180 können dabei die Strukturinformationen der Datenbasis nutzen, um Zugriffe auf die Datenbasis oder darin vorkommende Dokumente zu verbessern und/oder zu beschleunigen. Beispiel für solche Strukturen sind Schlüssel-Wert-Kombinationen 240/250, Tabellen, CSV Dateien, Bäume und ähnliches.

Geospezifische Dokumente 999, oder auch Geodaten 999 oder Geopositionen 999, sind daher Dokumente/Patientenmetadaten 100, die mit einer oder mehreren Geoinformationen 999 assoziiert sind oder diese selbst beinhalten. Solche Geoinformationen 999 können z. B. Längengrad, Breitengrad und Höhe sein. Zusätzlich zu der reinen Koordinatenabbildung können Geodaten 999 natürlich auch Region oder Länderbezeichner oder Länder ISO codes oder ähnliches darstellen, um grobgranularer mit den Daten zu arbeiten.

Es ist zu beachten, dass die Erfindung 180 auf das Nutzen von strukturierten Laborwerten 110 und strukturierten Patientenmetadaten 100 ausgelegt ist. Patientenmetadaten 100 können dabei zum Einen manuell erfasst werden aber auch auf mehrere Geräte oder Netzwerke verteilt sein. Auszeichnend für Patientenmetadaten 100 und Labordaten ist, dass diese strukturiert und für Maschinenlesbarkeit ausgelegt vorliegen.

Sofern die Laborwerte 110 oder Patientenmetadaten 100 nicht strukturiert vorliegen, benutzt die Erfindung 180 den Stand der Technik, um die unstrukturierten Dokumente aufgrund von künstlicher Intelligenz, Regeln 90 oder anderen Algorithmen in strukturierte Dokumente 800 zu überführen. Beispielsweise kann es vorkommen, dass ein Patient vorherige Untersuchungsergebnisse und andere Daten auf seinem Smartphone als Bilder oder in anderen unstrukturierten Datenformaten abgelegt hat, die dann als Patientenmetadaten 100 übertragen werden sollen. Um diese nun in ein strukturiertes Datenformat zu überführen, kann die Erfindung 180 z. B. Object Character Recognition (OCR) oder/und andere Bilderkennungsmechanismen und/oder Strukturierungsregeln 90 und/oder Machine Learning Verfahren nutzen, um strukturierte maschinenlesbare Patientenmetadaten 100/800 und Laborwerte 110 zu ermitteln, die dann als Dokumente 800 in der Erfindung 180 weiterverarbeitet werden. Hierbei ist zu beachten, dass bei solchen Strukturieren auch Standortdaten 999 aus Bildern von Laborwerten 110 extrahiert werden können, um den Standort 999 der Untersuchung besonders vorteilhaft strukturiert zu den Patientenmetadaten 100 hinzuzufügen. Die Erkennung der Standorte 999 aus Bildern funktioniert dabei wie folgt: Smartphones speichern beim Fotografieren oftmals Metadaten über den Standort 999, wie z. B. durch das Exchangeable Image File Format (Exif). In diesen wird dann oftmals der Standort 999 in der Bilddatei als Metadatum vermerkt. Somit können fotografierte Dokumente einfach auf Exif Metadaten überprüft werden und können dann als Patientenmetadaten 100 in die weitere Verarbeitung einfließen.

Durch die strukturierten Dokumente 800 kann z. B. auf Werte 250 in den Patientenmetadaten 100 und Laborwerten 110 über einen Index oder Schlüssel 240 zugegriffen werden. Selbstverständlich kann auch auf die gleiche Art und Weise auf Subschlüssel 240 zugegriffen werden.

Durch ihre Eigenschaft als Dokumente können Patientenmetadaten 100 beispielsweise als Resultat von Untersuchungen in externen Geräten, in Cloud Speichern und Profilen, Social Media Profilen und zugehörigen Apps, Elektronischen Patientenakten oder Verwaltungsprogrammen existieren. Weiterhin ist es möglich, dass Untersuchungsdaten, wie zuvor bestimmte Laborwerte 110, auch über einen eine CD,Scanner oder eine Fotografie eingelesen oder über ein Computernetzwerk 220 als Patientenmetadaten 100 und/oder Laborwerte 110 übertragen werden. Ebenfalls lassen sich besonders vorteilhaft Fitness Tracker Daten oder Daten von einer Smartwatch, wie z. B. regelmäßige Pulsmessungen oder Daten über die tägliche Aktivität als Patientenmetadaten 100 verwenden.

Da Patientenmetadaten 100 oftmals aus einer Vielzahl von Quellen stammen, da z. B. Standortdaten 999 nicht nur auf dem Smartphone des Benutzers zu findensind (z. B. wird die Standorthistorie 999 eines Benutzers durch das Smartphone Betriebssystem/Apps oftmals an die Hersteller Cloud übertragen). Aus diesem Grund können Patientenmetadaten 100 auch aus den verschiedensten Quellen im Internet geladen oder mit diesen Daten angereichert werden. Neben Standorthistoriendaten 999 können beispielsweise auch Daten aus dem Social Media Profil eines Benutzers oder dessen Fitness Tracker App Service übertragen werden. Selbstverständlich ist es auch möglich direkt den Standort 999 des Smartphones vom Smartphone über ein Computernetzwerk 220 zu übertragen/freizugeben, damit diese als oder in Patientenmetadaten 100 verwendet werden können.

Sofern Patientenmetadaten 100 keinen Standort 999 beinhalten, kann zusätzlich zu diesen auch ein Standort 999 eines Diagnosegerätes, der Erfindung 180 oder einer Untersuchung als zusätzlicher oder alternativer Standort 999 verwendet werden. Um z. B. den Standort 999 einer Untersuchung, eines Diagnosegerätes oder der Erfindung 180 zu bestimmen, kann GPS Hardware, durch Funk-Positionsbestimmung (https://de.wikipedia.org/wiki/WLAN-basierte_Ortung) oder durch IP-Adressen-Auflösung bestimmt werden (eine IP-Adresse eines Landes, einer Organisation und/oder bestimmten Benutzern) zugeordnet werden. Anschließend kann auf den Standort 999 geschlossen werden. Siehe auch
https://de.wikipedia.org/wiki/Geolokationssoftware) .

Oftmals existiert auch das Problem, dass keine historischen Standortinformationen 999 für die die Patientenmetadaten 100 vorhanden sind. Daher können diese Standortinformationen 999 auch besonders vorteilhaft aus Bildern von einem Patienten Smartphone extrahiert werden, indem die Bilder in der Galerie des Patientensmartphones nach Standorten 999 durchsucht werden, indem wie bei den Laborwerten 110 Exif Metadaten oder ein ähnliches Format analysiert werden und aus diesen Daten zusammen mit dem Aufnahmedatum ein Standortverlauf 999 erstellt wird. Alternativ hierzu können natürlich auch Standortverläufe 999 aus Social Media Profilen extrahiert werden.

Weiterhin können Geoposition/Geodaten 999 auch noch einfach manuell ausgewählt werden. Somit kann eine Adresse in eine Geoposition 999 ausgewählt werden und diese wird dann als Geoposition 999 weiterverwendet. Generell ist es auch wiederum möglich, dass eine Geoposition 999 in ein Land oder eine Region aufgelöst ist und dies dann als Patientenmetadatum 100 verwendet wird.

Patientenmetadaten 100, Patientendaten 100 (120) oder auch Patienteninformationen (120) oder Patientenmetadaten 100 genannt sind somit Daten von oder über einen spezifischen Patienten selbst. Diese Patientenmetadaten 100 sind somit individuelle Informationen, da diese spezifisch einem Individuum zugeordnet werden können, von welchem auch Laborwerte 110 bestimmt werden oder wurden.

Ebenfalls können Patientenmetadaten 100 auch Daten von, zu dem Individuum, vergleichbaren Patienten oder dessen Familienmitgliedern enthalten, damit besonders verstärkend auch Erbkrankheiten beachtet werden können. Patientenmetadaten 100 können insbesondere auch zuvor bestimmte Laborwerte 110, wie zuvor diagnostizierte Krankheiten oder Leiden eines Patienten sein. Ebenfalls ist es möglich, dass Patientenmetadaten 100 Leiden, Krankheiten oder und potentiell vererbbare Krankheiten von Familienmitgliedern enthalten. Zusätzlich ist es möglich, dass Vermutungen über Diagnosen in den Patienteninformationen enthalten sind. Letztendlich werden als Patientenmetadaten 100 alle personenbezogenen Informationen eines Patienten bezeichnet, die in einer medizinischen Einrichtung aufgenommen, verarbeitet und archiviert werden. Diese Daten werden im Sinne der Erfindung 180 elektronisch als Dokumente erfasst und können über Computernetzwerke 220 übertragen werden. Besonders vorteilhaft können die Daten im Sinne der Erfindung 180 auch anonymisiert, pseudonymisiert oder rein Teile der Daten übertragen werden, um Datensparsamkeit und Datenschutz zu gewährleisten. Beispielsweise kann somit nur ein Teil der Patientenmetadaten 100, der für eine Bewertung wichtig ist, oder auch nicht personenzuordenbar ist, ist übertragen werden.

Beispiele für Patientenmetadaten 100 sind: Alter, Geschlecht, Telefonnummer, Email, Behandlungsdaten, Diagnosen, Untersuchungsergebnisse mit Untersuchungsdatum, Therapiemaßnahmen, Beschwerden, Symptome, Wohnort, Beruf, Aufenthalte der letzten Wochen, Ernahrungsdetails, Aktivitäten der letzten Tage oder Wochen, Statur, Gewicht, Größe, Hobbys, Schwangerschaftswunsch, Schwangerschaft, Schwangerschaftsdauer, Schwangerschaftsanfang, Fehlgeburten, Lebensort, Patientenstammdaten, GPS Daten, Sportlichkeit, Blutdruck, Standort 999 der Analyse und Diagnosen von Eltern und Geschwister nebst deren Alter bei der Diagnose.

Besonders vorteilhaft können Patientenmetadaten 100 auch durch Laborwerte 110 dynamisch bestimmt und für die Bewertung anderer Laborwerte 110 verwendet werden. Beispielsweise ist es möglich durch HCG Werte die Dauer einer Schwangerschaft zu ermitteln (https://mamibees.de/hcg-tabelle/), die dann bei der fortfolgenden Bewertung als weitere Patientenmetadaten 100 dient.

Laborwerte 110 sind z. B. Blutwerte wie diese: https://www.gesundheit.gv.at/labor/Laborwerte 110/inhalt), das Biomarker oder weitere Eigenschaften im Blut oder Urin misst. Ein Laborwert 250 (110) ist das quantitative oder qualitative Ergebnis einer Untersuchung von Körpermaterialien (z. B. Blut, Urin) bzw. im erweiterten Sinn auch der Messung von Körperfunktionen. Oftmals bedeutet Laborwert 110 das Ergebnis einer bestimmten Blut- oder Urinuntersuchung oder ein Blutbild, das sich aus verschiedenen Laborwerten 110 zusammensetzt. Beispiele für Laborwerte 110 sind:
Blutbild Gerinnungsdiagnostik (z. B. INR-Wert, PTT), Serumeiweiße (z. B. Albumin), Serumelektrolyte (z. B. Serumnatrium), Organmarker Leberwerte (z. B. Gamma-GT), Nierenwerte (z. B. Kreatinin, Cystatin C), Bauchspeicheldrüsenmarker (z. B. Lipase), Muskelmarker (z. B. CK), Herzmuskelmarker (z. B. CK-MB), Stoffwechselmarker, Kohlenhydratstoffwechselmarker (z. B. Blutzucker, HbA1c), Knochenstoffwechselmarker (z. B. alkalische Phosphatase, Calcium, Phosphat), Fettstoffwechselmarker (z. B. Cholesterin, Triglyceride, HDL-Cholesterin, LDL-Cholesterin), Eisenstoffwechselmarker (z. B. Serum-Ferritin), Diabetesmarker (Glucose), Entzündungsparameter (z. B. CRP, IgG), Tumormarker und Infektionsserologie, z. B. Hepatitis-Serologie. Weitere Beispiel für Laborwerte 110 finden sich im Internet:
https://www.gesundheit.gv.at/labor/Laborwerte 110/inhalt oder https://www.apotheken-umschau.de/diagnose/Laborwerte 110/

Im Folgenden werden bevorzugte Ausführungsformen der Erfindung 180 offenbart, welche nach Ansicht der Erfinder den besten Arten und Weisen der Durchführung der Erfindung 180 entsprechen. Diese Ausführungen dienen als Beispiel, um das Verständnis zu erhöhen und sind daher nur als exemplarische, beispielhafte und nicht beschränkende Ausführungen zu verstehen, die in allen möglichen Kombinationen der verschiedenen Ausführungen oder Teile derer realisiert werden können. Zwar wird die Erfindung 180 im Zusammenhang mit diesen spezifischen Ausführungsbeispielen beschrieben, jedoch sei darauf hingewiesen, dass es nicht beabsichtigt ist, die Erfindung 180 auf die beschriebenen Ausführungsbeispiele zu beschränken. Im Gegenteil ist es beabsichtigt Alternativen, Modifikationen und Äquivalente abzudecken, die in den, durch die beigefügten Ansprüche, definierten Rahmen der Erfindung 180 fallen.

Weiterhin werden im Folgenden zahlreiche Details beschrieben, um ein umfassendes Verständnis der Erfindung 180 zu garantieren. Aus diesem Grund sei es angemerkt, dass die vorliegende Erfindung 180 auch unter der Auslassung dieser Details durchführbar ist. Ebenfalls werden die verschiedenen Ausführungsformen zwar als eigenständige Erweiterungen und/oder Spezialisierungen der Erfindung 180 beschrieben, jedoch ist dies zum Zwecke der Verständlichkeit, um die vorliegende Erfindung 180 nicht zu verunklaren. Daher können Merkmale der verschiedenen Ausführungsformen der Erfindung 180 beliebig mit anderen Ausführungsformen der Erfindung 180 kombiniert und getauscht werden.

### Figuren:

Beispiele für verschiedenen Ausführungsformen der Erfindung 180 oder Teile derer sind in den Figuren gegeben. Wir zeigen hierbei Figuren und Beispiele, dass ein mit dem Stand der Technik vertrauter Experte durch diese Beschreibung die Ausführungen der Erfindung 180 mit den in den Ansprüchen definierten Merkmalen umsetzen kann.

### Fig. 1

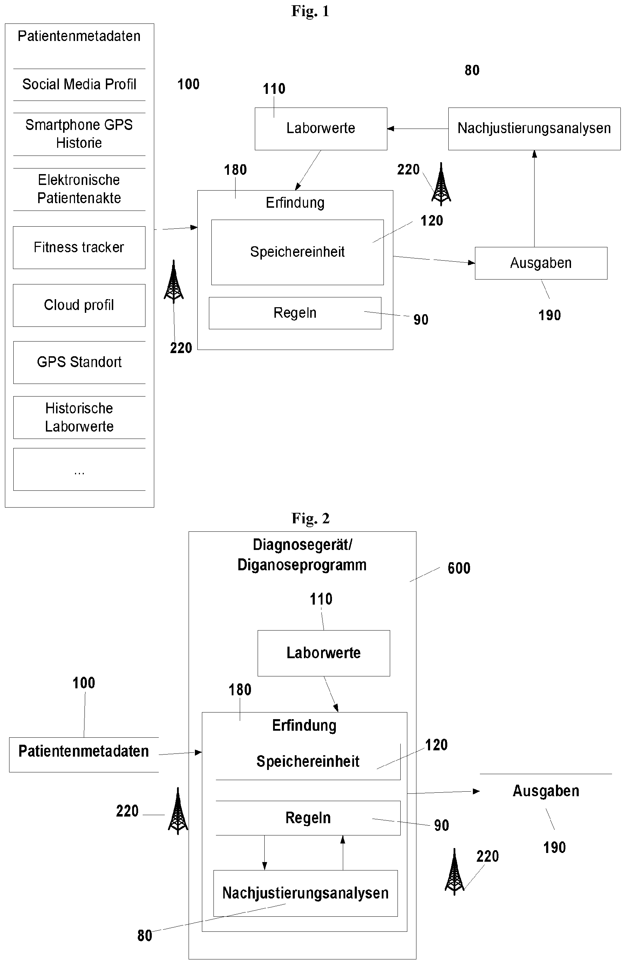

zeigt eine Ausführungsform der Erfindung 180. Hier werden Patientenmetadaten 100 als Dokumente übermittelt. Dies kann z. B. über ein Computernetzwerk 220 oder andere Eingaben geschehen. Ebenfalls werden Laborwerte 110 an die Erfindung 180 übertragen, was z. B. auch in der Form von Dokumenten über ein Computernetzwerk 220 geschehen kann. Alternativ hierzu dient die Erfindung 180 direkt als Erweiterung eines Laborgeräts (siehe hierzu als ein Beispiel Fig. 1b - Diagnosegerät), welches Werte bestimmt.

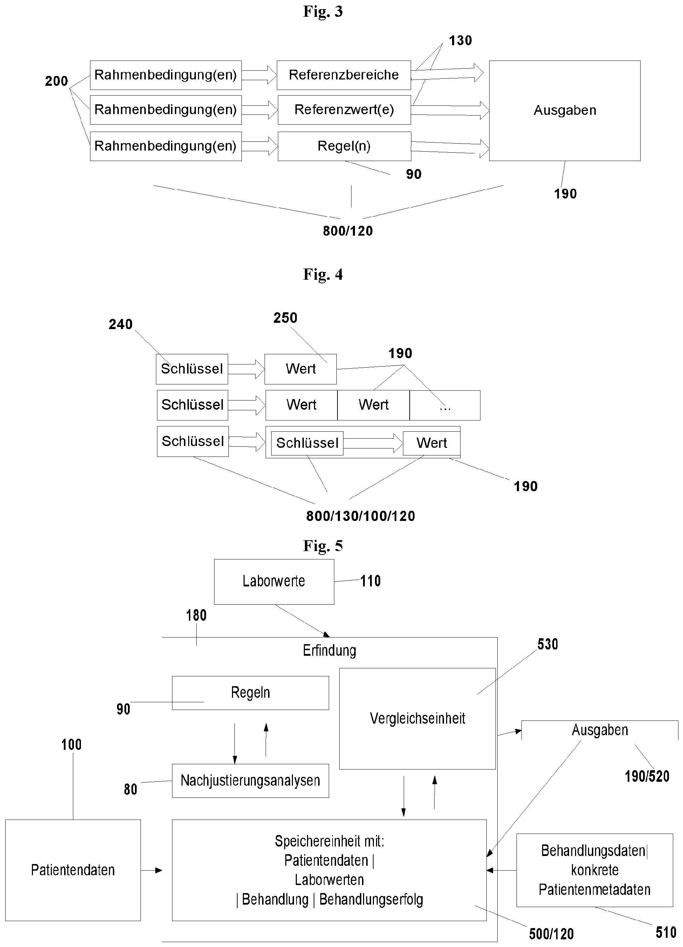

In einer Speichereinheit 120 der Erfindung 180 sind Referenzbereiche 130 von Laborwerten 110 Rahmenbedingungen 200 zugeordnet und mit potentiellen Ausgaben 190 wie Hinweisen, Handlungsempfehlungen, Handlungsanweisungen, Nachjustierungsanalyseanweisungen 80 und/oder Diagnoseempfehlungen und weiteren Daten verknüpft. Eine solche Zuordnung kann z. B. durch Schlüssel-Wert-Kombinationen 240/250 (Fig. 3, Fig. 4..) oder andere ähnliche Datenstrukturen 800 vorgenommen werden.

Beispielsweise werden hierdurch in der Speichereinheit 120 Referenzbereiche 130 Rahmenbedingungen 200 wie Schwangerschaft, Altersgruppen, Georegionen, Diagnosen, Geschlecht und Ähnlichem zugeordnet. Für eine Referenzwertüber- oder Unterschreitung können dann besonders vorteilhaft Hinweise 190, Handlungsempfehlungen, Handlungsanweisungen, Nachjustierungsanalyseanweisungen 80 und/oder Diagnoseempfehlungen in der Datenbank abgelegt sein.

Die Erfindung 180 nutzt Patientenmetadaten 100, um übereinstimmende oder ähnliche Rahmenbedingungen 200 aus der Speichereinheit 120 aufzudecken, um dann die Laborwerte 110 mit den zugeordneten Referenzbereichen 130 zu vergleichen. Dies kann z. B. durch einen Schlüssel-Wert-Vergleich 240/250 geschehen (Beispiel von Schlüssel-Wert-Datenstrukturen 240/250/800 Fig 3, Fig 4). Hierbei verwendet die Erfindung 180 Schlüssel 240 in den Patientenmetadaten 100 und zugehörige Werte und vergleicht diese mit übereinstimmenden oder ähnlichen Werten in den Rahmenbedingungen 200. Hierbei können auch kompliziertere Vergleiche 250 durch Regeln 90 oder Fuzzy Logic vorgenommen werden.

Dadurch, dass ähnliche oder übereinstimmende Daten in den Rahmenbedingungen 200 in der Speichereinheit 120 aufgedeckt werden, sind somit die zugehörigen Referenzwerte 130 für Laborwerte 110 aufgedeckt. Solche Referenzwerte 130 können z. B. eine physikalische Kenngröße sein. Beispiele für Referenzwerte 130 und Rahmenbedingungen 200 verdeutlichen den Inhalt der Speichereinheit 120 am besten:
Ein Referenzwert 130 kann z. B. 2,02 - 2,60 mmol/l für einen Calcium Laborwert 110 sein. Kombiniert mit Rahmenbedingungen 200/240 würde dann der Speicherinhalt wie folgt aussehen:
Rahmenbedingung 200/240: Neugeborene: Referenzwert: 1,75 - 2,70 mmol/l Calcium
Rahmenbedingung 200/240: Säuglinge, Kinder: 2,0 5 - 2,70 mmol/l Calcium
Rahmenbedingung 200/240: Erwachsene: 2,02 - 2,60 mmol/l Calcium

Eine andere Rahmenbedingung 200 wie das Lebensalter kann z. B. auch eine Schwangerschaft sein. z. B.:
Rahmenbedingung 200/240: Erstes Trimester 250: Calcium 110/240 2.2 - 2.65 mmol/L 250;
Rahmenbedingung 200/240: zweites Trimester 250: Calcium 110/240 2.05 - 2.25 mmol/L 250;
Rahmenbedingung 200/240: Drittes Trimester 250:Calcium 110/240 2.05 - 2.43 mmol/L 250;
Optimalerweise können bei der Rahmenbedingung 200 noch Zusatzinformationen in der Speichereinheit 120 vorhanden sein. Beispielsweise:
Rahmenbedingung: Trimester 200/240: 1 (250):
TSH Wert 110/240 über 2.5 mU/L 250; Zusatzinformationen 250: Leicht erhöhtes Risiko für Fehlgeburten;
TSH Wert 110/240 über 4.5 mU/L 250; Zusatzinformationen 250: Stark erhöhtes Risiko für Fehlgeburten.

Ähnliche weitere Referenzen 130 für Werte die äquivalent für die Schwangerschaft verwendet werden können sind: 1. Lockitch G. Handbook of Diagnostic Biochemistry and Hematology in Normal Pregnancy. Boca Raton:CRC, 1993. 2. Abbassi-Ghanavati M, Greer LG, Cunningham FG. Pregnancy and laboratory studies: a reference table for clinicians. Obstet Gynecol. 2009 Dec;114(6):1326-31. PMID:19935037 oder auf
http://www.perinatology.com/

Weiterhin verfügt die Erfindung 180 über eine Regeleinheit 90, die besonders optimal Referenzbereiche 130 beurteilt. Beispielsweise können durch die Anwendung von Regeln 90 Auswirkungen von der Sportlichkeit eines Patienten auf Referenzbereiche 130 berücksichtigt werden, indem zuvor aufgedeckte Referenzbereiche 130 verkleinert oder vergrößert werden. Beispiele hierfür sind in der Zusammenfassung der Erfindung 180 gegeben.

Durch das Nutzen verschiedener Laborwerte 110 zugleich mit Regeln 90 können besonders vorteilhaft Bewertungen vorgenommen werden und dadurch Ausgaben 190 in der Form von Empfehlungen für Diagnosen oder Diagnosehinweise abgeleitet werden. Dadurch, dass die Bewertungen Patientenmetadaten 100 beachten, ist es zudem möglich, dass der besondere Hintergrund eines oder mehrerer Patienten berücksichtigt wird und zumindest eine Diagnose oder ein Diagnosehinweis erstellt werden kann

Besonders optimal verfügt die Erfindung 180 noch über eine spezielle Einheit für Nachjustierungsanalysen 80.

Sofern bei einem Vergleich mit in der Speichereinheit 120 hinterlegten Laborwerten 110 und Rahmenbedingungen 200 Laborwerte 110 Patientenmetadaten 100 und Handlungsanweisungen 190 hinterlegt sind, die es empfehlen einen weiteren Laborwert 110 zu bestimmen, kann dieses fortfolgend und ohne menschliches Eingreifen durchgeführt werden. Um diese Variante der Erfindung 180 für direkte Nachjustierungsanalysen 80 bestmöglich einzusetzen, kann die Erfindung 180 direkt auf einem Laboranalysegerät 600 eingesetzt werden, damit besonders dynamisch bei der ursprünglichen Bestimmung eines Wertes sofort alle anhängigen Laborwerte 110 bei Referenzwertabweichungen bestimmt werden.

Ein Beispiel ist ein Calcium Laborwert 110/240 von 2.8 mmol/L 250 und folgende Referenzdaten 130:
Calcium 240: > 2.65 mmol/L 250; Handlungsanweisung: Bestimmen von Vitamin D Wert
Vitamin-D 25(OH) 240: <50 nmol/l 250; Handlungsanweisung: Bestimmen von Parathormon-Wert Parathormon 240: >67 ng/1250: Ausgabe: Starker Verdacht auf Hyperparathyreoidismus

Auch rein mit dem Alter als Patientenmetadaten 100 lassen sich Laborwerte 110 besser bewerten. Menschen im höheren Lebensalter haben einen tendenziellen höheren TSH-Wert 250 (https://www.endo-bochum.de/endokrinologie/tsh-spiegel-thyreoida-stimulierendes-hormon/). An das Alter angepasste Referenzwerte 130 können gemeinsam mit dem Alter in den Patientenmetadaten 100 nun verwendet werden, um besonders vorteilhaft Bewertungen durchzuführen, ob der Laborwert 110 für das Alter außerhalb des Referenzbereichs 130 liegt.

Das Resultat von einer oder mehreren Bewertungen wird dann verwendet werden, um Empfehlungen 190 (oder auch Hinweise) für Diagnose oder Diagnosehinweise zu erstellen. Solche Empfehlungen für Diagnosen können dabei die Bewertungen und Erklärungen beinhalten, wie es zu den Bewertungen kam und welche Laborwerte 110 dafür relevant sind.

Zum Beispiel könnte ein solcher Hinweis bei den Patientenmetadaten 100, Schwangerschaft im 02. Trimester mit dem Laborwert 110 Calcium 240: 2.4 mmol/L 250 wie folgt aussehen:
"Verdacht auf Hyperkalzämie, da Schwangerschaft-Labor-Referenzwert 02. Trimester: 8.2 - 9 mg/dL /2.05 - 2.25 mmol/L." Besonders vorteilhaft kann hierbei ein Hinweis 190 durch Annotation direkt an einem Laborwert 110 im Blutbild erfolgen oder die relevanten Laborwerte 110 aufgelistet werden.

Zusätzlich können interne Handlungsanweisungen 190 für automatisierte weitere Tests existieren, wie z. B.: Laborwert 110 von Vitamin D und Parathormon (PTH) bestimmen.

Bei einer solchen Ausgabe können dann auch noch Hinweise erfolgen, wie zum Beispiel eine Ausgabe 190, dass eine Hyperkalzämie (Diagnose) von Ursache Hyperparathyreoidismus (Ursachenhinweis) kommen kann oder auch, dass eine Hyperkalzämie oftmals zu Schwangerschaftsabbrüchen führt (Diagnosehinweis). Ebenso kann ein Hinweis 190, welche weiteren Laborwerte 110 getestet werden sollten, direkt erfolgen (Handlungsempfehlung), sofern nicht eine automatisierte Handlungsanweisung 190 diese Laborwerte 110 schon getestet hat (wobei diese automatisiert getesteten Werte dann auch mit Hinweisen auf die automatisierte Testung versehen sein können). Eine solche "manuelle" Ausgabe 190 als Handlungsempfehlung kann im konkreten Fall dann die Empfehlung sein erneut eine Blutprobe zu nehmen und dabei Vitamin D und Parathormon (PTH) zu bestimmen.

### Fig. 2

zeigt eine Ausführungsform der Erfindung 180, bei der diese direkt in einem Diagnosegerät oder einem Diagnoseprogramm eingesetzt wird. Dies kann zum Beispiel ein Gerät in einem Labor sein, das Blut und Urinproben analysiert. Wie zu sehen ist, können hier direkt im Gerät Nachjustierungsanalysen 80 durchgeführt werden. Oftmals kann hierdurch die Notwendigkeit entfallen Daten über ein Computernetzwerk 220 zu übertragen.

### Fig. 2

zeigt eine generell Datenstruktur 800 wie Rahmenbedingungen 200 mit Referenzbereichen 130 und Regeln 90 in der Speichereinheit 120 abgelegt sein können oder verarbeitet werden. Zusätzlich ist noch gezeigt, wie Ausgaben 190 in dieser Datenstruktur 800 besonders vorteilhaft verknüpft sein können.

### Fig. 3

zeigt wie eine Datenstruktur 800 Rahmenbedingungen 200 mit Referenzbereichen 130 und Ausgaben 190 in der Speichereinheit der Erfindung 120 verknüpft und abgelegt sein können. Hierbei ist auch zu sehen, dass Rahmenbedingungen 200 auch Regeln 90 und zugehörigen Ausgaben 190 zugeordnet sein können. Dies ermöglicht es, dass die Erfindung 180 besonders flexibel und effizient Laborwerte 110 und Patientenmetadaten 100 auf Rahmenbedingungen 200 abbilden und zugehörige Referenzwerte 130 aufdecken kann.

### Fig. 4

zeigt eine generelle schlüsselbasierte Datenstruktur 800, wie sie in vielen Bereichen dieser Erfindung 180 eingesetzt werden kann. Beispielsweise können hierdurch die Daten in der internen Speichereinheit 120 strukturiert werden. Ebenfalls kann selbige Datenstruktur 800 auch für Patientenmetadaten 100, Ausgaben 190, Regeln 80 und Nachjustierungsanalysen 80 verwendet werden.

Somit können jede Art von Patientenmetadaten 100 mit Schlüssel 240 und Werten 250 besonders vorteilhaft strukturiert werden. Ebenfalls kann selbige Struktur für Laborwerte 110 und deren Bezeichner verwendet werden. Wie zu sehen ist, können auch Werte 250 wiederum selbst weitere Objekte wie z. B. wiederum Schlüssel-Wert-Kombinationen 240/250 sein. Zusätzlich kann natürlich ein Schlüssel 240 auch mit mehreren Werten 250 assoziiert sein.

Durch die generelle Schlüssel-Wert-Struktur 240/250 können somit besonders effizient Schlüsselwerte 240 aus den verschiedensten Datenquellen aufeinander abgebildet und durch Regeln 90 verarbeitet werden.

### Fig. 5

zeigt eine besonders vorteilhafte Ausführung der Erfindung 180, durch die konkrete Behandlungsfälle als Behandlungsbeispiele/fälle 510/500/120 und nicht nur als Referenzwerte 130 aufgedeckt werden. Um dies zu bewirken, verfügt die Erfindung 180 über spezielle Erweiterungen:
a. Zumindest eine Speichereinheit 120/500 in der konkrete Patientenmetadaten 100 mitsamt ihrer konkreten Laborwerten 110, Diagnosen, durchgeführten Behandlungen und dem zugehörigen Behandlungsresultat 510, vorliegend sind.
b. Zumindest eine Vergleichseinheit 530, die es ermöglicht Patientenmetadaten 100 und Laborwerte 110 zu vergleichen.

Besonders vorteilhaft kommen noch eine Erweiterung/Schnittstelle 510 zum Einsatz, die es ermöglicht Daten über Behandlungen und Behandlungsverläufe mitsamt konkreten Patientenmetadaten 100 in der Speichereinheit 120 zu speichern.

Optional kann eine weitere Erweiterung die Daten anonymisieren und /oder pseudonymisieren und/oder in dieser Form speichern.

Wesentlich in dieser Ausprägungsform ist es, dass es möglich ist besonders vorteilhaft konkrete Patientenmetadaten 100 zu vergleichen (D.h. Patientenmetadaten 100 eines Patienten A mit Patientenmetadaten 100 eines anderen Patienten B und nicht nur abstrakte Daten wie in den Rahmenbedingungen 200). Dies ermöglicht es im ersten Schritt Patientenmetadaten 100 eines konkreten Patienten A mitsamt dessen Laborwerten 110 zu empfangen und dann in der Speichereinheit 120/400 ähnliche Patienten bzw. deren Daten durch Regeln 90 aufzufinden oder die Datenbasis 500/120 danach zu filtern.

Konkrete Patientenmetadaten 100 und erfolgte Behandlungen 510 sind dabei Datensätze von und über einen Patienten, woraus zu entnehmen ist, wie dieser und mit welchem Behandlungserfolg dieser behandelt wurde. Hierbei kann auch in den strukturierten Daten (die Datenstruktur 800 kann analog zu Fig. 4 sein) vermerkt sein, welche konkreten Werte ausschlaggebend für eine Diagnose waren.

Um die konkreten Patientenmetadaten 100 in der Speichereinheit 120/500 abzulegen, kann die Speichereinheit 120/500 als Datenbank, Big Data Cluster, NoSQL Datenbank oder durch Polyglott Persistenz mit einer logischen Abfrageschnittstelle realisiert werden.

Besonders vorteilhaft können dabei auch Ausgaben 190 und Empfehlungen/Hinweise 190, die auch von anderen Ausführungsformen der Erfindung 180 stammen, mitsamt der erfolgten Behandlung besonders optimal in der Speichereinheit 120/500 abgelegt werden, damit diese Fälle bei weiteren Diagnosen besonders vorteilhaft verwendet werden können.

Das Auffinden ähnlicher Patienten kann dabei durch Regeln 90, wie z. B. Datenbankabfragen von Werten mit Bedingungen 200 geschehen, aber es können auch weitaus komplexere technische Verfahren eingesetzt werden.

Zum Beispiel kann eine Vergleichseinheit 530 diese gesamte Ähnlichkeit zwischen Patientenmetadaten 100 und Laborwerten 110 berechnen. Dies kann z. B. durchgeführt werden, indem einzelne Laborwerte 110 und Patientenmetadaten 100 wie Schilddrüsenwerte, Alter, Geschlecht, Diagnosen und Sportlichkeit durch Regeln 90 verglichen werden. Ein Einzel- und Gesamtvergleich kann dabei in unterschiedlichster Weise berechnet werden: Beispielsweise können Histogramme von allen Patientenmetadaten 100 in der Speichereinheit 120/500 über Wertebereiche 250 einzelner Laborwerte 110 besonders vorteilhaft Standardabweichungen darstellen.

Zu Histogrammen hinzu können alternativ oder zusätzlich Ähnlichkeiten auf eine andere Art und Weise berechnet werden. Beispielsweise ist es möglich bei jeder Paarung (konkrete Werte Patient A - konkrete Werte Patient B) einfach die Beträge der Wertdifferenzen zu summieren und ein hoher Wert = unähnlich. Oder bei jeder Paarung kann die Quadrate der Differenzen summiert werden und durch eine Abweichung einzelner Werte die Ähnlichkeit verringert werden. Zusätzlich oder alternativ können Gewichtungen der Abweichungen durchgeführt werden, indem diese mit einem Faktor multipliziert werden und somit gewichtet in die gesamte Ähnlichkeitsbewertung einfließen. Dann können alle Abweichungen zusammen aufsummiert werden, um eine komplette Ähnlichkeitsbewertung zwischen zwei Datensätzen von Patienten zu erzeugen. Es sei hierbei noch erwähnt, dass optimalerweise auch mehrere unabhängige Ähnlichkeitsbewertungen durchgeführt werden können, um besonders vorteilhaft mehrere Ähnlichkeitsfaktoren abzubilden.

Diese Fälle /Datensätze, die zwischen Patient A und B übereinstimmen, können nach Ähnlichkeitsbewertungen sortiert, diejenigen mit der höchsten Übereinstimmung z. B. Top 10, optional anonymisiert, und als Ausgabe der Erfindung 180 verwendet werden. Dies ermöglicht es, dass eine breite Datenbasis von vielen Patientenbehandlungen schnell und effizient gefiltert werden. Einem Arzt stehen somit vor dem Durchführen einer Diagnose ähnliche Referenzfälle zur Verfügung. Durch reine menschliche Arbeitskraft wäre dies nicht möglich einfach ähnliche Fälle aufzudecken, da insbesondere die Ähnlichkeitsvergleiche nicht ohne Weiteres möglich wären.

Durch die verschiedenen Ausführungen der Erfindung 180 wird es auch klar, dass nicht alle Einheiten und Verfahren der Erfindung 180 zwangsläufig direkt auf einem System operieren müssen. Vielmehr ist es möglich, dass Einheiten teilweise auf Systemen, die über ein Computernetzwerk 220 verbunden sind, ausgelagert sein können.

### Fig. 6

zeigt eine Ausführungsform der Erfindung 180 als Verfahren.

Im ersten Schritt 900 wird zumindest ein Referenzwert 130 (oder auch Referenzbereich 130) zumindest eines Laborwerts 110 und zumindest eine Zuordnung zu einer Rahmenbedingung 200 gespeichert. Hierbei können Referenzwerte 130 zusätzlich zu Rahmenbedingungen 200 auch mit Ausgaben 190 wie Hinweisen, Risiken, Handlungsempfehlungen, Handlungsanweisungen und Ursachenhinweisen, Nachjustierungsanweisungen 80 und/oder Diagnoseempfehlungen verknüpft sein.

In einem weiteren Schritt 910 werden Dokumente mit Patientenmetadaten 100 mitsamt von zumindest einem Laborwert 110 über zumindest ein Computernetzwerk 220 übertragen. Z. B. werden Laborwerte 110 wie TSH, PTH, Thrombozyten-Zählung etc. mitsamt den Patientenmetadaten 100, wie z. B. Patientenalter und dessen Diagnosen, übertragen.

Im nächsten Schritt 920 werden die zuvor übertragenen Patientenmetadaten 100 genutzt, um zumindest einen Referenzwert 130 aufgrund von übereinstimmenden Rahmenbedingungen 200 aufzudecken. Z. B. wird das Geschlecht und das Alter verwendet, um Referenzwerte 130 aus dem ersten Schritt 900 aufzudecken, die mit den Patienteninformationen übereinstimmen.

In einem nächsten optionalen Schritt 930 werden nun die zuvor aufgedeckten Referenzwerte 130 aufgrund zumindest einer Regel 90 angepasst. Eine solche Regel 90 kann z. B. sein, bei äquatornahen oder südlichen Geodaten 999 in den Patientenmetadaten 100 den unteren Vitamin-D Referenzwertbereich 130 nach oben zu verschieben. Ein weiteres Beispiel ist es, in den Patientenmetadaten 100 sportlich definierten Personen den unteren TSH Referenzwertbereich 130 um einen 0,5 Punkt nach oben zu verschieben. Dies ermöglicht es besonders zielgenau Referenzbereiche 130 anzupassen.

Im nächsten Schritt 940 wird ein Laborwert 110 nun mit den zuvor in Schritt 920 oder 930 bestimmten Referenzwerte/Referenzwertbereichen 130 verglichen und eingeordnet, ob der Laborwert 110 sich noch in den Referenzwertgrenzen 130 befindet.

In einem weiteren Schritt 950 wird das Ergebnis des Vergleichs aus Schritt 940 optional mit den angewandten Justierungen (verschieben der Referenzwertbereiche) aus vorherigen Schritten ausgegeben, indem dieses z. B. visualisiert, in einem Dokument gespeichert und/oder über ein Computernetzwerk 220 übertragen wird.

Durch diese Ausgabe 190 wird besonders vorteilhaft die Information gewonnen, welche genaue Patientenmetadaten 100 für die Referenzwertbeurteilung 130 beachtet wurde. Sofern die Referenzwertbeurteilung 130 z. B. durch eine Geoposition 999 mitbeeinflusst wurde, kann somit besonders vorteilhaft auch die geologische Ursache als Hinweis 190 bei der Ausgabe 190 mit übermittelt werden.

Wie zu sehen ist, verhält sich das Verfahren analog zu der Realisierung der Erfindung 180 als Apparat. Aus diesem Grund können selbstverständlich alle Merkmale der Erfindung 180 in eines oder mehrere Verfahren umgewandelt werden und umgekehrt. Daher sind die in dieser Anmeldung beschriebenen Merkmale der Erfindung 180 auch als Merkmale eines oder mehrerer Verfahren zu sehen und Merkmale von Verfahren können auch Merkmale von Apparaten sein.

### Fig. 7

zeigt ein Beispiel von Geodaten 999 oder einem Standortverlauf 999, wie er als Patientenmetainformationen 100 in der Erfindung 180 verwendet werden kann. Wie zu sehen ist, kann eine oder mehrere Geopositionen 999 dazu verwendet werden, um zu bestimmen welche Rahmenbedingungen 200 für Patientenmetadaten 100 beachtet werden müssen. Dies ist besonders vorteilhaft, da somit durch Regeln 90 besser auf örtlich beschränkte Referenzwerte 130 oder vorherrschende Krankheiten bei Laborwerten 110 geachtet werden kann.

## Patentansprüche

1. Eine Erfindung als System oder Anordnungen (180) zum Erstellen von Ausgaben (190) aufgrund von Dokumenten, die Laborwerte (110) und Patientenmetainformationen (100) beinhalten, wobei ein System oder eine Anordnung zumindest folgende Merkmale aufweisen:
a. Zumindest eine Speichereinheit (120), die zumindest eine Rahmenbedingung (200) RB und hierzu zumindest einen verknüpften Referenzbereich (130) von Laborwerten (110) enthält;
b. Zumindest ein Laborwert (110) und zumindest ein Patientenmetadatum (100), das über zumindest ein Computernetzwerk (220) übertragen wird;
c. Zumindest ein übertragenes Patientenmetadatum (100), verwendet wird um zumindest einen Referenzbereich (130) R aus einer Speichereinheit (120) zu bestimmen, indem die Übereinstimmung zumindest einer Rahmenbedingung (200) RB und dem Patientenmetadatum (100) geprüft wird;
d. Zumindest eine Bewertung B von zumindest einem Laborwert (110) aus b. unter der Einbeziehung von zumindest einem Referenzbereich (130) R;
e. Ausgabe zumindest einer Bewertung B.

2. Ein System oder Anordnung nach Anspruch 1, wobei
f. die Laborwerte (110) in zumindest einer Datenstruktur (800) vorliegen, die definiert ist als Schlüssel (240) und Wert (250); und
g. bei zumindest einer Bewertung B der Schlüssel (240) eines Laborwertes (110) aus b. dazu genutzt wird einen passenden Referenzbereich (130) mit dem äquivalenten Schlüssel (240) im Speicher (120) zu vergleichen.

3. Ein System oder Anordnung nach einem der vorhergehenden Ansprüche, wobei die Referenzbereiche (130) R in a. in zumindest einer Datenstruktur (800) definiert sind, die zumindest als Schlüssel (240) und Referenzwertbereich (130) definiert ist.

4. Ein System oder Anordnung nach einem der vorhergehenden Ansprüche, wobei zumindest eine Regel (90) verwendet wird, um zumindest einen Referenzbereich (130) R anzupassen.

5. Ein System oder Anordnung nach einem der vorhergehenden Ansprüche, wobei zumindest eine Regel (90) verwendet wird, um eine Mehrzahl von Referenzabweichungen (130) zu bewerten.

6. Ein System oder Anordnung nach einem der vorhergehenden Ansprüche, wobei
h. die Patientenmetadaten (110) in zumindest einer Datenstruktur (800) vorliegen, die definiert ist als Schlüssel (240) und Wert (250); und
i. in c.
I. zumindest ein Schlüssel (240) aus einem Patientenmetadatum (110) mit zumindest einem Schlüssel (240) aus einer Rahmenbedingung (200) verglichen wird; und
II. zumindest ein Wert (250) aus einem Patientenmetadatum (110) mit zumindest einem Wert oder Wertebereich in den Rahmenbedingungen (200) verglichen wird; und
j. aufgrund der Vergleiche in i. Referenzwerte (130) für Laborwerte (110) aufgedeckt und fortfolgend bei der Bewertung B verwendet werden.

7. Ein System oder Anordnung nach einem der vorhergehenden Ansprüche, wobei zumindest eine Bewertung B zumindest eine Ermittlung zumindest eines weiteren Laborwertes (110) zur Folge (80) hat dessen nachfolgende Bewertung B' automatisch eine vorherige Ausgabe (190) verbessert.

8. Ein System oder Anordnung nach einem der vorhergehenden Ansprüche, wobei die Patientenmetadaten (100) zumindest ein Geodatum (999) enthalten und zumindest ein Geodatum (999) bei zumindest einer Bewertung beachtet wird.

9. Ein System oder Anordnung nach Anspruch 8, wobei zumindest ein Geodatum (999) in zumindest einem mobilen Endgerät aufgezeichnet und über zumindest ein Computernetzwerk (220) übertragen wird.

10. Ein System oder Anordnung nach einem der vorhergehenden Ansprüche, wobei eine Bewertung B zumindest eines Laborwertes (110) zumindest ein Risiko (190) in einer Schwangerschaft enthält.

11. Ein System oder Anordnung nach einem der vorhergehenden Ansprüche, wobei die Patientenmetadaten (100) einen potentiellen Schwangerschaftswunsch beinhalten und die Bewertung B zumindest eines Laborwertes (110) Fruchtbarkeitseinschränkungen beurteilt.

12. Ein System oder Anordnung nach einem der vorhergehenden Ansprüche, wobei eine Ausgabe (190) zumindest Hyperparathyrodismus indiziert und zumindest eine Regel (90), die Laborwerte (110) von Calcium, Vitamin D und das Parathormon verarbeitet.

13. Ein System oder Anordnung nach einem der vorhergehenden Ansprüche, wobei die Erfindung
k. Patientenmetadaten (100) und Laborwerte (110) von einem Patienten A empfängt; und
l. über eine Speichereinheit (120/500) verfügt, in der
I. zumindest ein konkretes Patientenmetadatum (100/510) und zugehöriger Laborwert (110/510) und
II. zumindest ein zugehöriger Behandlungserfolg (510) von einem oder mehreren weiteren Patienten B gespeichert sind; und
m. Daten von einem oder mehreren Patienten B mit Daten von Patient A verglichen werden und ähnliche Patientenmetadaten (100) und Laborwerte (110) von zumindest einem Patienten B zu den Daten von A bestimmt werden, wobei
I. Abweichungen der Datensätze von Patienten A und B als Wertabweichungen aufgrund von jeweils einzelnen Werten (110/240/250) berechnet werden und
II. die Gesamtheit verschiedener Wertabweichungen eine Ähnlichkeitsdistanz ausdrückt; und
n. zumindest ein ähnlicher Datensatz von Patient B mitsamt dem Behandlungserfolg (520/190) ausgegeben wird.

14. Ein Verfahren für das Erstellen von Ausgaben (190) in der Form von Hinweisen, Risiken, Handlungsempfehlungen, Handlungsanweisungen und Ursachenhinweisen (190) aufgrund von Dokumenten, die Laborwerte (110) und Patientenmetadaten (100) beinhalten, wobei das Verfahren zumindest die folgenden Schritte aufweist:
a. Speichern zumindest eines Referenzwertes (130) bestehend aus Schlüssel (240) und Wertbereich (250) eines Laborwertes (110) mitsamt zumindest einer Zuordnung zu zumindest einer Rahmenbedingung (200);
b. Übertragung zumindest eines Patientenmetadatums (100) mitsamt zumindest einem Laborwert (110), bestehend aus Schlüssel (240) und Wert (250), über zumindest ein Computernetzwerk (220);
c. Aufdecken von zumindest einem gespeicherten Referenzwert (130) aus a. unter dem Vergleich von
I. zumindest einem Patientenmetadatum (100) aus b. mit zumindest einer Rahmenbedingung (200); und
II. zumindest einem Laborwert (110) Schlüssel (240) aus b. mit zumindest einem Referenzwert Schlüssel (240) aus a.;
d. Vergleich von zumindest einem Laborwert (110) Wert (250) aus b. mit zumindest einem in c. bestimmten Referenzwert (130);
e. Zumindest eine Ausgabe (190) aufgrund zumindest eines Ergebnisses eines Vergleiches aus d.

15. Ein Computerprogramm, das Instruktionen aufweist, um ein Verfahren nach Anspruch 14 auszuführen.
